(19) Europäisches Patentamt
European Patent Office
Office européen des brevets

(11) EP 2 224 898 B1

(12) **EUROPEAN PATENT SPECIFICATION**

(45) Date of publication and mention of the grant of the patent:
**15.08.2018 Bulletin 2018/33**

(21) Application number: **08855629.5**

(22) Date of filing: **20.11.2008**

(51) Int Cl.:
***A61K 8/49*** *(2006.01)* ***A61Q 17/04*** *(2006.01)*
***A61K 8/37*** *(2006.01)*

(86) International application number:
**PCT/EP2008/065916**

(87) International publication number:
**WO 2009/068469 (04.06.2009 Gazette 2009/23)**

(54) **GRINDING AIDS FOR MICRONIZED ORGANIC UV ABSORBERS**

SCHLEIFMITTEL FÜR MIKRONISIERTE ORGANISCHE UV-LICHT-ABSORPTIONSMITTEL

ASSISTANCES ABRASIVES POUR ABSORBEURS D'UV ORGANIQUES MICRONISÉS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.11.2007 EP 07121894**

(43) Date of publication of application:
**08.09.2010 Bulletin 2010/36**

(73) Proprietor: **BASF SE**
**67056 Ludwigshafen am Rhein (DE)**

(72) Inventors:
- **MONGIAT, Sébastien**
**F-68510 Sierentz (FR)**
- **GRUMELARD, Julie**
**F-68330 Huningue (FR)**
- **EHLIS, Thomas**
**79100 Freiburg (DE)**

(74) Representative: **BASF IP Association**
**BASF SE**
**G-FLP-C006**
**67056 Ludwigshafen (DE)**

(56) References cited:
**EP-A- 0 893 119** **WO-A-03/063814**
**WO-A-2004/052837** **GB-A- 2 286 774**
**US-A- 5 980 872** **US-A1- 2004 191 191**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 224 898 B1

## Description

The present invention relates to a method for producing new formulations of UV absorbers and to their use in sunscreen compositions which, in turn, are useful, in particular, for the protection of human skin.
It has long been known that prolonged exposure to that UV radiation which reaches the surface of the earth can lead to the formation of erythemas or light dermatoses, as well as to an increased incidence of skin cancers or accelerated skin aging.
Various sunscreen formulations have been proposed which include a material which is intended to counteract UV radiation, thereby inhibiting the said undesired effects on the skin.
A great number of compounds has been proposed for use as UV protectants in sunscreen formulations, especially soluble organic UV absorbers and insoluble micronised inorganic compounds, in particular zinc oxide and titanium dioxide.
The high specific weight of insoluble inorganic compounds, such as zinc oxide and titanium dioxide leads to a reduced stability of formulations containing them. Moreover, such inorganic compounds have been claimed to generate toxic radicals under the influence of light ("Redox Mechanisms in Heterogeneous Photocatalysis", Serpone et al, Electrochemistry in Colloids and Dispersions, Editors Mackay and Texter, VCH Publishers Inc., NewYork 1992).
Micronised, insoluble organic UV absorbers, when used in sunscreen formulations, provide excellent UV protection and have a high SPF rating. Moreover, micronised, insoluble organic UV absorbers show no tendency, under the influence of light, to generate radicals which could damage or sensitise human skin.
Accordingly, the present invention provides a method of preparing a composition, comprising (a) a micronised insoluble organic UV absorber, as defined in claim 1, which method comprises grinding the insoluble organic UV absorber, in coarse particle form, in a grinding apparatus, in the presence of a grinding aid (b) selected from the groups (b1) sodium lauroyl lactylate or (b2) isocetyl isostearate, and/or glycol oleate Most preferred in the method of the present invention is the micronized insoluble UV absorber of the formula

(7) 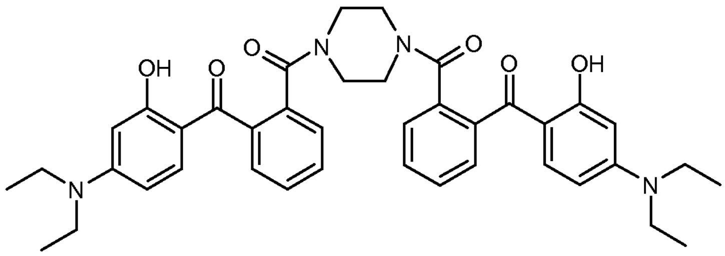

wherein
The sparingly soluble organic compounds which are used in the present invention are present in the micronized state and are preferably prepared by wet-milling processes.
As milling apparatus for the preparation of the sparingly soluble micronised organic compounds there may be used, for example, a jet mill, ball mill, vibratory mill or hammer mill, preferably a high-speed mixing mill. Even more preferable mills are modern ball mills; manufacturers of these types of mill are, for example, Netzsch (LMZ mill), Drais (DCP-Viscoflow or Cosmo), Bühler AG (centrifugal mills) or Bachhofer.
The insoluble organic UV absorber used in the present invention is preferably micronized in the presence of a grinding aid selected from (b1) sodium lauroyl lactylate or (b2) isocetyl isostearate, and/or glycol oleate. Preferrably the grinding aid is used in a concentration of 0.1 to 20% by weight, preferably 1 to 15 % b.w. based on the total weight of the UV protection composition.
Rheology modifiers (component (c)) are optionally added to the UV protection composition which help to stabilize across the time such composition.
Examples for rheology modifiers are synthetic polymers, natural polymers and their derivatives, mineral polymers etc., but also according to their ionic character such as anionic, cationic, nonionic or amphoteric as listed in the Table below:

| Table 1a: Natural thickeners<br>Most of them are derived from the Polysaccharides category | |
|---|---|
| RM 1 | Cellulose gum such as cross-linked or not Sodium Carboxymethylcellulose...or |

(continued)

| Table 2b: Mineral thickeners<br>Most of them are derived from smectite clays and silica derivatives | |
|---|---|
| RM 12 | Aluminum Silicates or Bentonites or Montmorillonites such as Magnesium Aluminum Silicates (Veegum range from R.T.Vanderbilt) and Quaternized compounds such as Stearalkonium Bentonite |
| RM 13 | Magnesium Silicates or Hectorites such as Bentone Series (from Elementis Specialties) and Quaternized compounds such as Disteardimonium Hectorite (to disperse in lipophilic media) |
| RM 14 | Magnesium sodium Fluorosilicate or modified Mica |
| RM 15 | Synthetic layered Silicates; similar structure to Hectorites; Sodium Magnesium |
| | Silicates (Laponite range from Solvay) |
| RM 16 | Fumed Silicas such as Aerosil range from Degussa |

| Table 2c: Synthetic Rheology modifiers<br>Poly(acrylic acid) PAA and its copolymers: within such structure, it can be incorporated ester groups, with hydrophilic character such as 2-Hydroxyethyl Methacrylate etc. | |
|---|---|
| RM 17 | Carbomer or crosslinked polyacrylic acid polymer such as Carbopol Ultrez 10, Carbopol ETD2001, Carbopol ETD2050 from Noveon Inc |
| RM 18 | Sodium polyacrylate (Cosmedia SP from Cognis), Acrylates copolymer (Carbopol Aqua SF-1 from Noveon Inc.), Acrylates/acrylamides Coplymer (Noveon EC-1 from Noveon Inc.) |
| RM 19 | Hydroxyethyl/Acrylate/Sodium Acryloyldimethyl Taurate copolymer (Simulgel NS or EG from Seppic); combination with Tinosorb M claimed in PCA N°161 Nov.2001 |
| RM 20 | Ammonium Polyacrylates (Simulgel A from Seppic) |
| =>"Hydro Swelling Droplets" concept | |
| RM 21 | - Glyceryl Polyacrylates (e.g.,Hispagel 100) or Polymethacrylates (e.g., Lubrajel range from ISP Corp.) |
| RM 22 | Poly(Acrylamide) PAAm and its copolymers; copolymers of ammonium acrylate and acrylamide; copolymers of AAam with long hydrophobic chain and acrylates |
| RM 23 | Poly(Ethylene oxide) PEO and Poly (Propylene oxide) PPO and their copolymers; these are block terpolymers of EO and PO with the structure ABA or BAB; A: PEO with good water solubility B: PPO with limited water solubility |
| RM 24 | Poly(VinylPyrrolidone)PVP homopoplymers or Poly(VinylPyrrolidone)/Vinyl Acetate coplymers |
| RM 25 | Poly (vinylalcohol) PVA |
| RM 26 | VA/Crotonates copolymer Poly(vinylacetate)/Crotonic acid or VA/Crotonates/Vinyl Neodecanoate copolymer |
| RM 27 | Ethylene/VinylAcetate copolymer such as A.C.coplymer400 (Allied-Signal) |
| RM 28 | PVM/MA copolymers and their esterified derivatives such Ethyl, Isopropyl or Butyl esters |
| RM 29 | PVM/MA Decadiene Crosspolymer; copolymer of methyl vinyl ether/ Maleic Anhydric (PVM/MA) crosslinked with 1,9-decadiene |
| RM 30 | Polyethylene resins such as PEG-2M to PEG-9M (RITA Corp.) |

(continued)

| Table 2c: Synthetic Rheology modifiers<br>Poly(acrylic acid) PAA and its copolymers: within such structure, it can be incorporated ester groups, with hydrophilic character such as 2-Hydroxyethyl Methacrylate etc. | |
|---|---|
| RM 31 | polysiloxanes and copolymers; copolymers of polysiloxanes and other blocks such as PEO blocks |
| RM 32 | PEG-modified materials, the most commonly used class of non ionic thickeners with the following basic structure: $R(OCH_2\,CH_2)_n\,OH$, werein R is the fatty moiety, like fatty alcohol, glyceryl ester, propylene glycol ester or carboxylic acid; for example; PEG-150 Distearate; these thickeners are not susceptible to hydrolysis and offer better viscosity stability under a broad range of pH and temperature profiles |
| RM 33 | Trihydroxystearin or Glycol Tri-(12-Hydroxystearate) |
| RM 34 | Glyceryl Tribehenate such as Syncrowax HRS-C from Croda |

| Table 2d: Phospholipid derivatives | |
|---|---|
| RM 35 | Alkylated Phosphatidyl Choline forming fluid lamellar assembly as the stable liquid crystalline phase of general formula:<br>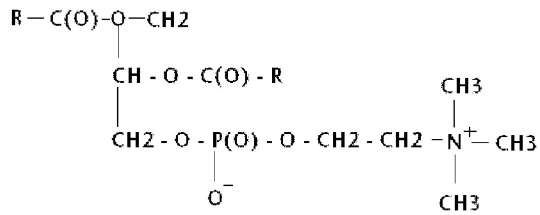<br>wherein R is $C_2$-$C_4$alkyl |
| RM 36 | Phosphobetaines (amphoteric ingredients); alkylamido Phosphobetaine of gerneral formula<br>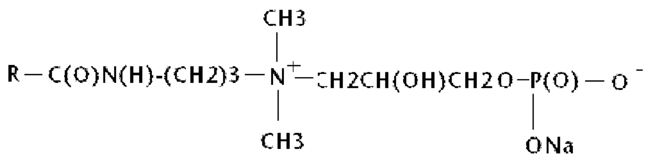<br>wherein R is $C_2$-$C_{14}$alkyl |
| RM 37 | Alkyl Phosphate Quaternary compounds of general formula<br>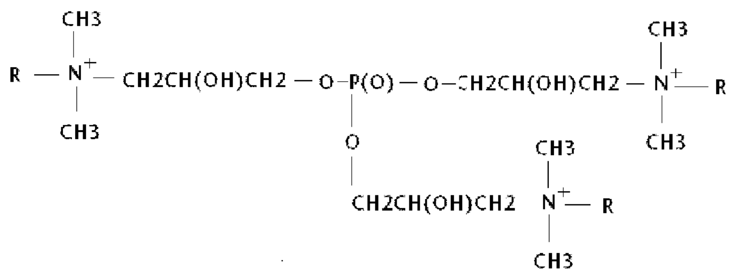<br>wherein R is $C_2$-$C_{14}$alkyl |

Most preferred rheology modifier (c) is Xanthan Gum, amorphous Silica or modified Starch.

Any known process suitable for the preparation of microparticles can be used for the preparation of the micronised UV absorbers, for example:

- wet-milling (low-viscosity micronisation process for pumpable dispersions), with a hard grinding medium, for example zirconium silicate balls in a ball mill, and a protective surfactant or a protective polymer in water or in a suitable

organic solvent;

- wet-kneading (high-viscosity micronisation process for non-pumpable pastes) using a continuous or discontinuous (batch) kneader. For a wet-kneading process, a solvent (water or cosmetically acceptable oils), a grinding aid (surfactant, emulsifier) and a polymeric grinding aid may be used.

Both processes may be used preferably.

- spray-drying from a suitable solvent, for example aqueous suspensions or suspensions containing organic solvents, or true solutions in water, ethanol, dichloroethane, toluene or N-methylpyrrolidone etc..
- by expansion according to the RESS process (Rapid Expansion of Supercritical Solutions) of supercritical fluids (e.g. $CO_2$) in which the UV filter or filters is/are dissolved, or the expansion of liquid carbon dioxide together with a solution of one or more UV filters in a suitable organic solvent;
- by reprecipitation from suitable solvents, including supercritical fluids (GASR process = Gas Anti-Solvent Recrystallisation / PCA process = Precipitation with Compressed Anti-solvents).

As milling apparatus for the preparation of the micronised organic UV absorbers there may be used, for example, a jet mill, ball mill, vibratory mill or hammer mill, preferably a high-speed mixing mill. Even more preferable mills are modern ball mills; manufacturers of these types of mill are, for example, Netzsch (LMZ mill), Drais (DCP-Viscoflow or Cosmo), Bühler AG (centrifugal mills) or Bachhofer. The grinding is preferably carried out with a grinding aid. Examples of kneading apparatus for the preparation of the micronised organic UV absorbers are typical sigma-blade batch kneaders but also serial batch kneaders (IKA-Werke) or continuous kneaders (Continua from Werner und Pfleiderer). The following are examples of preparation of micronized UV protection, however, not according to the claimed invention since (b1) sodium lauroyl lactylate or (b2) isocetyl isostearate, and/or glycol oleate are not used a grinding aids, but are present in the dispersions A1,A2 and A12:

For each example of micronized UV protection dispersion, the manufacturing process is operated as following; Compound of formula (7), (5) or (3) respectively (where (5) or (3) are not claimed) are milled together with zirconium silicate bells (diameter 0.1 to 4 mm) as grinding aids, the dispersing agent (as described inside examples A1 to A19) and the continuous phase, containing water, polyol and preservative system, in a ball mill (as described previously) to a mean particle size of $d_{50}$ from 100nm to 170nm. After the micropigment dispersion of UV absorber of formula (7), (5) or (3) respectively is obtained, the formulator incorporate the thickening agent according to the concentration mentioned in examples A1 to A19.

| Example A1: Dispersion 1 | | | | | | | | | |
|---|---|---|---|---|---|---|---|---|---|
| | A | B | C | D | E | F | G | H | I |
| | % w/w | % w/w | % w/w | % w/w | % w/w | % w/w | % w/w | % w/w | % w/w |
| Sparingly soluble micronized substance | | | | | | | | | |
| Compound of formula (7) | 35 | 50 | 65 | | | | | | |
| Compound of formula (5) | | | | 35 | 50 | 65 | | | |
| Compound of formula (3) | | | | | | | 35 | 50 | 65 |
| Dispersing agent | | | | | | | | | |
| Sodium Lauroyl Lactylate | 5 | 7.5 | 10 | 5 | 7.5 | 10 | 5 | 7.5 | 10 |
| Thickening agent | | | | | | | | | |
| modified Starch | | | | 0.4 | 0.3 | 0.2 | | | |
| xanthan gum | 0.2 | 0.2 | 0.3 | | | | 0.2 | 0.3 | 0.15 |
| Continuous phase | | | | | | | | | |
| propylene glycol | 0.3 | 0.3 | 0.3 | 0.3 | 0.3 | 0.3 | 0.3 | 0.3 | 0.3 |

(continued)

| | | | | | | | | | |
|---|---|---|---|---|---|---|---|---|---|
| water | qs. 100 | qs. 100 | qs. 100 | qs. 100 | qs. 100 | qs. 100 | qs. 100 | qs. 100 | qs. 100 |
| Preservative system such as Diazolidinyl Urea and parabens | | | | 0.7 | 0.5 | 0.4 | | | 0.6 |
| Preservative system such as phenoxyethanol and parabens | 0.5 | | 0.6 | | | | 0.4 | | |

| Example A2: Dispersion 2 | | | | | | | | | |
|---|---|---|---|---|---|---|---|---|---|
| | A | B | C | D | E | F | G | H | I |
| | % w/w | % w/w | % w/w | % w/w | % w/w | % w/w | % w/w | % w/w | % w/w |
| Sparingly soluble micronized substance | | | | | | | | | |
| Compound of formula (7) | 50 | | | 50 | | | 40 | | |
| Compound of formula (5) | | 50 | | | 50 | | | 40 | |
| Compound of formula (3) | | | 50 | | | 50 | | | 40 |
| Dispersing agent | | | | | | | | | |
| Isocetyl isostearate | 5 | 5 | 5 | | | | 5 | 5 | |
| Glycol oleate | | | | 5 | 5 | 5 | | | 5 |
| Thickening agent | | | | | | | | | |
| xanthan gum | 0.2 | 0.2 | 0.15 | 0.2 | 0.3 | 0.15 | | | |
| amorphous Silica | | | | | | | 0.3 | | |
| modified Starch | | | | | | | | 0.3 | 0.4 |
| Continuous phase | | | | | | | | | |
| propylene glycol | 0.3 | 0.3 | 0.3 | 0.3 | 0.3 | 0.3 | 0.3 | 0.3 | 0.3 |
| water | qs. 100 | qs. 100 | qs. 100 | qs. 100 | qs. 100 | qs. 100 | qs. 100 | qs. 100 | qs. 100 |
| Preservative system such as Diazolidinyl Urea and parabens | | | | 0.7 | 0.5 | 0.4 | | | 0.6 |
| Preservative system such as phenoxyethanol and parabens | 0.5 | | 0.6 | | | | 0.4 | | |

| Example A12: Dispersion 12 | | | | | | | | | |
|---|---|---|---|---|---|---|---|---|---|
| | A | B | C | D | E | F | G | H | I |
| | % w/w | % w/w | % w/w | % w/w | % w/w | % w/w | % w/w | % w/w | % w/w |
| Sparingly soluble micronized substance | | | | | | | | | |
| Compound of formula (7) | 50 | | | 50 | | | 50 | | |
| Compound of formula (5) | | 50 | | | 50 | | | 50 | |

(continued)

| Example A12: Dispersion 12 | | | | | | | | | |
|---|---|---|---|---|---|---|---|---|---|
| | A | B | C | D | E | F | G | H | I |
| | % w/w | % w/w | % w/w | % w/w | % w/w | % w/w | % w/w | % w/w | % w/w |
| Sparingly soluble micronized substance | | | | | | | | | |
| Compound of formula (3) | | | 50 | | | 50 | | | 50 |
| Dispersing agent | | | | | | | | | |
| Sodium dicocoylethylene diamine PEG-15 sulfate and Sodium Lauroyl Lactylate | 5 | 5 | 5 | 3 | 4 | 2 | | | |
| Sodium decylglucosides and Hydroxypropyl sulfonate | | | | 2 | | 3 | 5 | 5 | 5 |
| Thickening agent | | | | | | | | | |
| xanthan gum | 0.2 | 0.2 | 0.2 | 0.2 | 0.2 | 0.2 | 0.2 | 0.2 | 0.2 |
| Continuous phase | | | | | | | | | |
| propylene glycol | 0.3 | 0.3 | 0.3 | 0.3 | 0.3 | 0.3 | 0.3 | 0.3 | 0.3 |
| water | qs. 100 | qs. 100 | qs. 100 | qs. 100 | qs. 100 | qs. 100 | qs. 100 | qs. 100 | qs. 100 |
| Preservative system such as Diazolidinyl Urea and parabens | | | | 0.7 | 0.5 | 0.4 | | | 0.6 |
| Preservative system such as phenoxyethanol and parabens | 0.5 | | 0.6 | | | | 0.4 | | |

Other preferred preparation of micronized UV protection dispersions are detailed as following, however, not according to the claimed invention since (b1) sodium lauroyl lactylate or (b2) isocetyl isostearate, and/or glycol oleate are not used a grinding aids, but are present in the dispersion 20: For each example of micronized UV protection dispersion, the manufacturing process is operated as following:

Compound of formula (7) is milled together with zirconium silicate bells (diameter 0.1 to 4 mm) as grinding aids, the dispersing agent (as described inside dispersions 20 to 34) and the continuous phase, containing water, simethicone and occasionally citric acid, in a ball mill (as described previously) to a mean particle size of $d_{50}$ from 100nm to 170nm. After the micropigment dispersion of UV absorber of formula (7 is obtained, the formulator incorporate the thickening agent (Xanthan Gum) according to the concentration mentioned in dispersions 20 to 34.

| Dispersion 20 | | | | | |
|---|---|---|---|---|---|
| | % w/w | pH | Extinction coef. $E_{1,1}$ | Particle size distribution | |
| | | | | d 50 | d 90 |
| Compound of formula (7) | 50 | 7 | 470 | 123 nm | 246 nm |
| Sodium dicocoylethylene diamine PEG-15 sulfate and Sodium Lauroyl Lactylate | 9 | | | | |
| Simethicone | 0.9 | | | | |
| Water | Qs 100 | | | | |
| Xanthan Gum | 0.1 | | | | |

| | | | | | |
|---|---|---|---|---|---|
| Water | Qs 100 | | | | |
| Xanthan Gum | 0.1 | | | | |

| | | | | | |
|---|---|---|---|---|---|
| | 100 | | | | |
| Xanthan Gum | 0.1 | | | | |

| | | | | | |
|---|---|---|---|---|---|
| | 100 | | | | |
| Xanthan Gum | 0.1 | | | | |

| | | | | |
|---|---|---|---|---|
| Water | Qs 100 | | | |
| Citric acid | qs | | | |

| | | | | | |
|---|---|---|---|---|---|
| Water | Qs 100 | | | | |
| Xanthan Gum | 0.1 | | | | |

The UV absorber is defined in claim 5. The UV absorber composition according to the present invention may comprise one or more than one additional UV absorbers as described in the Tables 3 and 4.

| Table 3: Suitable UV filter substances which can be additionally used with the UV absorbers according to the present invention |
|---|
| p-aminobenzoic acid derivatives, for example 4-dimethylaminobenzoic acid 2-ethylhexyl ester; |
| salicylic acid derivatives, for example salicylic acid 2-ethylhexyl ester; |
| benzophenone derivatives, for example 2-hydroxy-4-methoxybenzophenone and its 5-sulfonic acid derivative; |
| diphenylacrylates, for example 2-ethylhexyl 2-cyano-3,3-diphenylacrylate, and 3-(benzofuranyl) 2-cyanoacrylate; |
| 3-imidazol-4-ylacrylic acid and esters; |
| benzofuran derivatives, especially 2-(p-aminophenyl)benzofuran derivatives, described in EP-A-582 189, US-A-5 338 539, US-A-5 518 713 and EP-A-613 893; |
| polymeric UV absorbers, for example the benzylidene malonate derivatives described in EP-A-709 080; |
| cinnamic acid derivatives, for example the 4-methoxycinnamic acid 2-ethylhexyl ester and isoamyl ester or cinnamic acid derivatives described in US-A-5 601 811 and WO 97/00851; |
| camphor derivatives, for example 3-(4'-methyl)benzylidene-bornan-2-one, 3-benzylidene-bornan-2-one, N-[2(and 4)-2-oxyborn-3-ylidene-methyl)-benzyl]acrylamide polymer, 3-(4'-trimethylammonium)-benzylidene-bornan-2-one methyl sulfate, 3,3'-(1,4-phenylene-dimethine)-bis(7,7-dimethyl-2-oxo-bicyclo[2.2.1]heptane-1-methanesulfonic acid) and salts, 3-(4'-sulfo)benzylidene-bornan-2-one and salts; camphorbenzalkonium methosulfate; |
| hydroxyphenyltriazine compounds, for example 2-(4'-methoxyphenyl)-4,6-bis(2'-hydroxy-4'-n-octyloxyphenyl)-1,3,5-triazine; 2,4-bisf[4-(3-(2-propyloxy)-2-hydroxy-propyloxy)-2-hydroxy]-phenyl}-6-(4-methoxyphenyl)-1,3,5-triazine; 2,4-bis{[4-(2-ethyl-hexyloxy)-2-hydroxy]-phenyl}-6-[4-(2-methoxyethyl-carboxyl)-phenylamino]-1,3,5-triazine; 2,4-bis{[4-(tris(trimethylsilyloxy-silylpropyloxy)-2-hydroxy]-phenyl}-6-(4-methoxyphenyl)-1,3,5-triazine; 2,4-bis{[4-(2"-methylpropenyloxy)-2-hydroxy]-phenyl}-6-(4-methoxyphenyl)-1,3,5-triazine; 2,4-bis{[4-(1',1',1',3',5',5',5'-heptamethyltrisilyl-2"-methyl-propyloxy)-2-hydroxy]-phenyl}-6-(4-methoxyphenyl)-1,3,5-triazine; 2,4-bis{[4-(3-(2-propyloxy)-2-hydroxy-propyloxy)-2-hydroxy]-phenyl}-6-[4-ethylcarboxy)-phenylamino]-1,3,5-triazine; |

(continued)

| Table 3: Suitable UV filter substances which can be additionally used with the UV absorbers according to the present invention |
|---|
| physical sunscreens, coated or not coated, such as titanium dioxide, zinc oxide, iron oxides, mica, MnO, $Fe_2O_3$, $Ce_2O_3$, $Al_2O_3$, $ZrO_2$ (surface coatings: polymethylmethacrylate, methicone (methylhydrogenpolysiloxane as described in CAS 9004-73-3), dimethicone, isopropyl titanium triisostearate (as described in CAS 61417-49-0), metal soaps such as magnesium stearate (as described in CAS 4086-70-8), perfluoroalcohol phosphate such as $C_9$-$C_{15}$ fluoroalcohol phosphate (as described in CAS 74499-44-8; JP 5-86984; JP 4-330007)). The primary particle size is, on average, 15 nm - 35 nm and the particle size distribution is in the range 100 nm - 300 nm. |
| aminohydroxy-benzophenone derivatives disclosed in DE 100 11 317, EP 1 133 980 and EP 1 046 391 |
| phenyl-benzimidazole derivatives as disclosed in EP 1 167 358 |

| Table 4: Suitable UV filter substances and adjuvants which can be additionally used with the UV absorbers according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts; Mexoryl SL | 56039-58-8 |
| 11 | 1-[4-(1,1-dimethylethyl)phenyl]-3-(4-methoxyphenyl)propane-1,3-dione; avobenzone | 70356-09-1 |
| 12 | Methyl N,N,N-trimethyl-4-[(4,7,7-trimethyl-3-oxobicyclo[2,2,1]hept-2-ylidene)methyl] anilinium sulphate; Mexoryl SO | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; octocrylene | 6197-30-4 |
| 30 | 2-ethylhexyl 4-(dimethylamino)benzoate | 21245-02-3 |
| 31 | 2-ethylhexyl 4-methoxycinnamate; octyl methoxy cinnamate | 5466-77-3 |
| 32 | 2-ethylhexyl salicylate | 118-60-5 |
| 33 | Benzoic acid, 4, 4', 4"-(1, 3, 5- triazine-2, 4, 6- triyltriimino)tris(2-ethylhexyl)ester; 2,4,6-Trianilino-(p-carbo-2'-ethylhexyl-1'-oxi)-1,3,5-triazine; octyl triazone | 88122-99-0 |
| 34 | 4-aminobenzoic acid | 150-13-0 |
| 35 | Benzoic acid, 4-amino-, ethyl ester, polymer with oxirane | 113010-52-9 |

(continued)

| Table 4: Suitable UV filter substances and adjuvants which can be additionally used with the UV absorbers according to the present invention | | |
|---|---|---|
| No. | Chemical Name | CAS No. |
| 38 | 2-phenyl-1H-benzimidazole-5-sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl] phenyl]methyl]-, homopolymer | 147897-12-9 |
| 40 | Triethanolamine salicylate | 2174-16-5 |
| 41 | 3,3'-(1,4-phenylenedimethylene)bis[7,7-dimethyl-2-oxo-bicyclo[2.2.1]heptane-1 methanesulfonic acid]; Cibafast H | 90457-82-2 |
| 42 | Titanium dioxide | 13463-67-7 |
| 44 | Zinc oxide | 1314-13-2 |
| 46 | 1H-Benzimidazole-4,6-disulfonic acid, 2,2'-(1,4-phenylene)bis-, disodium salt | 180898-37-7 |
| 47 | Benzoic acid, 4,4'-[[6-[[4-[[(1,1-dimethylethyl)amino]carbonyl]phenyl]amino]1,3,5-triazine-2,4-diyl]diimino]bis-, bis(2-ethylhexyl)ester; diethylhexyl butamido triazone; Uvasorb HEB | 154702-15-5 |
| 48 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane; Mexoryl XL | 155633-54-8 |
| 49 | Dimethicodiethylbenzalmalonate; Polysilicone 15; Parsol SLX | 207574-74-1 |
| 50 | Benzenesulfonic acid, 3-(2H-benzotriazol-2-yl)-4-hydroxy-5-(1-methylpropyl)-, monosodium salt; Tinogard HS | 92484-48-5 |
| 51 | Benzoic acid, 2-[4-(diethylamino)-2-hydroxybenzoyl]-, hexyl ester; Uvinul a plus | 302776-68-7 |
| 52 | 1-Dodecanaminium, N-[3-[[4-(dimethylamino)benzoyl]amino]propyl]-N,N-dimethyl-, salt with 4-methylbenzenesulfonic acid (1:1); Escalol HP610 | 156679-41-3 |
| 53 | 1-Propanaminium, N,N,N-trimethyl-3-[(1-oxo-3-phenyl-2-propenyl)-amino]-, chloride | 177190-98-6 |
| 54 | 1H-Benzimidazole-4,6-disulfonic acid, 2,2'-(1,4-phenylene)bis- | 170864-82-1 |
| 55 | 1,3,5-Triazine, 2,4,6-tris(4-methoxyphenyl)- | 7753-12-0 |
| 56 | 1,3,5-Triazine, 2,4,6-tris[4-[(2-ethylhexyl)oxy]phenyl]- | 208114-14-1 |
| 57 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 58 | 2-Propenoic acid, 3-(1H-imidazol-4-yl)- | 104-98-3 |
| 59 | Benzoic acid, 2-hydroxy-, [4-(1-methylethyl)phenyl]methyl ester | 94134-93-7 |
| 60 | 1,2,3-Propanetriol, 1-(4-aminobenzoate); glyceryl PABA | 136-44-7 |
| 61 | Benzeneacetic acid, 3,4-dimethoxy-a-oxo- | 4732-70-1 |
| 62 | 2-Propenoic acid, 2-cyano-3,3-diphenyl-, ethyl ester | 5232-99-5 |
| 63 | Anthralinic acid, p-menth-3-yl ester | 134-09-8 |
| 64 | 2,2'-bis(1,4-phenyiene)-1H-benzimidazoie-4,6-disuiphonicacid mono sodium salt or Disodium phenyl dibenzimidazole tetrasulfonate or Neoheliopan AP | 349580-12-7, |
| 65 | 1,3,5-Triazine-2,4,6-triamine, N,N'-bis[4-[5-(1,1-dimethylpropyl)-2-benzoxazolyl] phenyl]-N''-(2-ethylhexyl)- or Uvasorb K2A | 288254-16-0 |
| 66 | Merocyanine derivatives as described in WO 2004006878 and in I PCOM000022279D | |

(continued)

| Table 4: Suitable UV filter substances and adjuvants which can be additionally used with the UV absorbers according to the present invention | | |
|---|---|---|
| No. | Chemical Name | CAS No. |
| 67 | 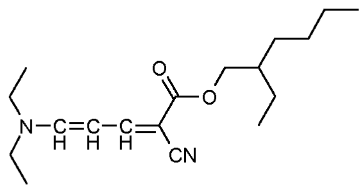 | |
| 68 | sterols (cholesterol, lanosterol, phytosterols), as described in WO0341675 | |
| 69 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 70 | alpha-lipoic-acid as described in DE 10229995 | |
| 71 | synthetic organic polymers as described in EP 1371358, [0033]-[0041] | |
| 72 | phyllosilicates as described in EP 1371357 [0034]-[0037] | |
| 73 | silica compounds as described in EP1371356, [0033]-[0041] | |
| 74 | inorganic particles as described in DE10138496 [0043]-[0055] | |
| 75 | latex particles as described in DE10138496 [0027]-[0040] | |
| 76 | 1H-Benzimidazole-4,6-disulfonic acid, 2,2'-(1,4-phenylene)bis-, disodium salt; Bisimidazylate; Neo Heliopan APC | 180898-37-7 |
| 77 | 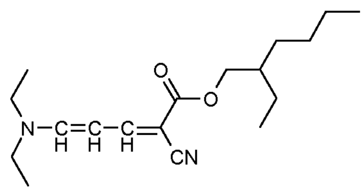 | |
| 78 | 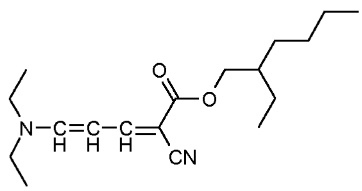 | |
| 79 | 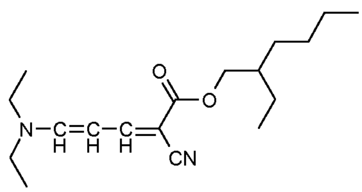 | |
| 80 | 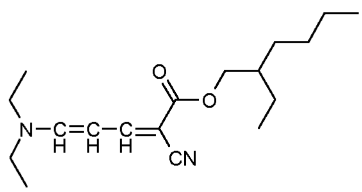 | |

(continued)

| Table 4: Suitable UV filter substances and adjuvants which can be additionally used with the UV absorbers according to the present invention | | |
|---|---|---|
| No. | Chemical Name | CAS No. |
| 83 | Di-2-ethylhexyl-3,5-dimethoxy-4-hydroxy-benzalmalonate (Oxynex ST, EMD Chemicals, as described in US 20040247536) | |

The cosmetic or pharmaceutical preparations may be, for example, creams, gels, lotions, alcoholic and aqueous/alcoholic solutions, emulsions, wax/fat compositions, stick preparations, powders or ointments. In addition to the above-mentioned UV filters, the cosmetic or pharmaceutical preparations may contain further adjuvants as described below.

As water- and oil-containing emulsions (e.g. W/O, O/W, O/W/O and W/O/W emulsions or microemulsions) the preparations contain, for example, from 0.1 to 30 % by weight, preferably from 0.1 to 15 % by weight and especially from 0.5 to 10 % by weight, based on the total weight of the composition, of one or more UV absorbers, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the invention may also contain one or one more additional compounds like fatty alcohols, esters of fatty acids, natural or synthetic triglycerides including glyceryl esters and derivatives, pearlescent waxes:hydrocarbon oils: silicones or siloxanes, organosubstituted superfatting agents, surfactantsconsistency regulators/thickeners and rheology modifiers, polymers, biogenic active ingredients, deodorising active ingredients, anti-dandruff agents, antioxidants, hydrotropic agents, preservatives and bacteria-inhibiting agents, perfume oils, colorants, polymeric beads or hollow spheres as spa enhancers.

Cosmetic or pharmaceutical preparations

Cosmetic or pharmaceutical formulations are contained in a wide variety of cosmetic preparations. There come into consideration, for example, especially the following preparations:

- skin-care preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;

- fragrance preparations, e.g. fragrances (eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.

Presentation forms

The final formulations listed may exist in a wide variety of presentation forms, for example:

- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition: from 0.01 to 5 % by weight of a UV absorber composition according to the invention, 12.0 % by weight of sodium laureth-2-sulfate, 4.0 % by weight of cocamidopropyl betaine, 3.0 % by weight of sodium chloride, and water ad 100%.

Other typical ingredients in such formulations are preservatives, bactericides and bacteriostatic agents, perfumes, dyes, pigments, thickening agents, moisturizing agents, humectants, fats, oils, waxes or other typical ingredients of cosmetic and personal care formulations such as alcohols, poly-alcohols, polymers, electrolytes, organic solvents, silicon derivatives, emollients, emulsifiers or emulsifying surfactants, surfactants, dispersing agents, antioxidants, anti-irritants and anti-inflammatory agents etc.

The cosmetic preparation according to the invention is distinguished by excellent protection of human skin against the damaging effect of sunlight.

Method to assess in vitro Sun Protection Factor measurement (SPF)

End-product application rate 1.4 mg/cm$^2$ on PMMA plates (Helioplates®)
UV Transmittance analysis with Labsphere UV-1000S Transmittance Analyser

$$SPF = \frac{\int_{290nm}^{400nm} E_\lambda \cdot S_\lambda \cdot d\lambda}{\int_{290nm}^{400nm} E_\lambda \cdot S_\lambda \cdot T_\lambda \cdot d\lambda}$$

wherein $E_\lambda$ = erythema action spectrum;$S_\lambda$ = solar spectral irradiance and $T_\lambda$ = spectral transmittance of the sample.

Method to assess in vitro UVA protection factor (UVA PF)

End-product application rate 1.2 mg/cm$^2$ on PMMA plates (Helioplates®)
UV Transmittance analysis with Labsphere UV-1000S Transmittance Analyser
Pre-irradiation step (to take the sun care product photostability into account) via a solar simulator as Atlas Suntest CPS+

$$\mathrm{PF\,UVA} = \frac{\sum_{320}^{400} \Delta\lambda}{\sum_{320}^{400} T_\lambda \cdot \Delta\lambda} = \frac{1}{T_\mathrm{m}}$$

Wherein $T_\lambda$ = sunscreen product transmittance at wave length $\lambda$ and $T_m$ = mean arithmetical value of Transmittance data in the UVA range.

B. Cosmetic formulations (Not claimed)

| Example B1: Emulsion high Protection | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Cyclomethicone | 7,00 | 7,00 | 7,00 | 7,00 | 7,00 | 7,00 | 7,00 | 7,00 | 7,00 |
| | Ethylhexyl Palmitate | 5,00 | 5,00 | 5,00 | 5,00 | 5,00 | 5,00 | 5,00 | 5,00 | 5,00 |
| | Glyceryl Stearate | 1,50 | 1,50 | 1,50 | 1,50 | 1,50 | 1,50 | 1,50 | 1,50 | 1,50 |
| | Octocrylene | 1,00 | 1,00 | 1,00 | 5,00 | 6,00 | 8,00 | 4,00 | 3,00 | 4,00 |
| | Ethylhexyl Triazone | 6,00 | 6,00 | 6,00 | 2,00 | 3,00 | 4,00 | 2,00 | 3,00 | 4,00 |
| | Potassium Cetyl Phosphate | 1,80 | 1,80 | 1,80 | 1,80 | 1,80 | 1,80 | 1,80 | 1,80 | 1,80 |
| | VP/Eicosene Copolymer | 0,60 | 0,60 | 0,60 | 0,60 | 0,60 | 0,60 | 0,60 | 0,60 | 0,60 |
| Part B | Organic micro-particulate UV absorber of formula (3), (5) or (7) | 2,00 | 4,00 | 6,00 | 8,00 | 10,00 | 14,00 | 16,00 | 18,00 | 20,00 |
| Part C | Aqua | Qsto 100 | Qsto 100 | Qsto 100 | Qsto 100 | Qs to 100 | Qs to 100 | Qs to 100 | Qs to 100 | Qs to 100 |
| | Acrylates/Palmeth-25 Acrylate | 0,25 | 0,25 | 0,25 | 0,25 | 0,25 | 0,25 | 0,25 | 0,25 | 0,25 |
| | Copolymer | | | | | | | | | |
| | Phenyl benzimidazole sulfonic acid | 1,00 | 2,00 | 3,00 | 1,00 | 2,00 | 3,00 | 2,00 | 2,00 | 2,00 |
| | Glycerin | 6,00 | 6,00 | 6,00 | 6,00 | 6,00 | 6,00 | 6,00 | 6,00 | 6,00 |
| | Disodium EDTA | 0,20 | 0,20 | 0,20 | 0,20 | 0,20 | 0,20 | 0,20 | 0,20 | 0,20 |

(continued)

| Example B1: Emulsion high Protection | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part D | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Tocopheryl Acetate | 0,30 | 0,30 | 0,30 | 0,30 | 0,30 | 0,30 | 0,30 | 0,30 | 0,30 |
| In vitro SPF measured according the method described | | 18,5 | 25,1 | 30,9 | 22,4 | 29,2 | 41,7 | 25,2 | 29 | 35,2 |
| UVA PF measured according the method described | | 2,5 | 3,3 | 4 | 5,2 | 5,9 | 7,4 | 7,4 | 7,8 | 8,4 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. The mixture is homogenized for 30 sec at 10000rpm.

| Example B2: Sun Milk | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | C12-15 Alkyl Benzoate | 4,00 | 4,00 | 4,00 | 4,00 | 4,00 | 4,00 | 4,00 | 4,00 | 4,00 |
| | Octocrylene | 7,50 | 7,50 | 7,50 | 7,50 | 7,50 | 7,50 | 7,50 | 7,50 | 7,50 |
| | Isohexadecane | 3,00 | 3,00 | 3,00 | 3,00 | 3,00 | 3,00 | 3,00 | 3,00 | 3,00 |
| | Cyclopentasiloxane | 4,00 | 4,00 | 4,00 | 4,00 | 4,00 | 4,00 | 4,00 | 4,00 | 4,00 |
| | Stearic Acid | 0,50 | 0,50 | 0,50 | 0,50 | 0,50 | 0,50 | 0,50 | 0,50 | 0,50 |
| | PEG-100 Stearate (and) Glyceryl Stearate | 0,80 | 0,80 | 0,80 | 0,80 | 0,80 | 0,80 | 0,80 | 0,80 | 0,80 |
| | Potassium Cetyl Phosphate | 1,30 | 1,30 | 1,30 | 1,30 | 1,30 | 1,30 | 1,30 | 1,30 | 1,30 |
| | Butyl Methoxydibenzoylmethane | 6,00 | 4,00 | 2,00 | 3,00 | 5,00 | 2,00 | 3,00 | 2,00 | 1,00 |
| | Ethylhexyl Methoxycinnamate | 6,00 | 5,00 | 4,00 | 6,00 | 5,00 | 4,00 | 3,00 | 4,00 | 3,00 |
| | Titanium Dioxide | 8,00 | 3,00 | 4,00 | 6,00 | 3,00 | 4,00 | 2,00 | 3,00 | 2,00 |
| | PVP/Eicosene Copolymer | 1,00 | 1,00 | 1,00 | 1,00 | 1,00 | 1,00 | 1,00 | 1,00 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | Qsto 100 | Qsto 100 | Qsto 100 | Qsto 100 | Qsto 100 | Qsto 100 | Qsto 100 | Qsto 100 | Qsto 100 |
| | Glycerin | 4,00 | 4,00 | 4,00 | 4,00 | 4,00 | 4,00 | 4,00 | 4,00 | 4,00 |

(continued)

| Example B2: Sun Milk | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Propylene Glycol | 5,00 | 5,00 | 5,00 | 5,00 | 5,00 | 5,00 | 5,00 | 5,00 | 5,00 |
| | Xanthan Gum | 0,20 | 0,20 | 0,20 | 0,20 | 0,20 | 0,20 | 0,20 | 0,20 | 0,20 |
| | Acrylates/C10-30 Alkyl Acrylate Cross polymer | 0,15 | 0,15 | 0,15 | 0,15 | 0,15 | 0,15 | 0,15 | 0,15 | 0,15 |
| | Disodium EDTA | 0,15 | 0,15 | 0,15 | 0,15 | 0,15 | 0,15 | 0,15 | 0,15 | 0,15 |
| Part D | Triethanolamine | Qs | Qs | Qs | Qs | Qs | Qs | Qs | Qs | Qs |
| | Dimethicone | 0,40 | 0,40 | 0,40 | 0,40 | 0,40 | 0,40 | 0,40 | 0,40 | 0,40 |
| | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Tocopheryl Acetate | 0,90 | 0,90 | 0,90 | 0,90 | 0,90 | 0,90 | 0,90 | 0,90 | 0,90 |
| In vitro SPF measured according the method described | | 40,9 | 32,2 | 38,2 | 49,7 | 45,5 | 58,1 | 57,7 | 65,6 | 66,6 |
| UVA PF measured according the method described | | 9,8 | 9,3 | 10,1 | 11,6 | 11,5 | 12,9 | 14,1 | 13,9 | 13,2 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then the pH is checked and adjusted with triethanolamine.

| Example B3: Sun Milk | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | C12-15 Alkyl Benzoate | 4,00 | 4,00 | 4,00 | 4,00 | 4,00 | 4,00 | 4,00 | 4,00 | 4,00 |
| | Butylen Glycol Dicaprylate/ Dicaprate | 3,00 | 3,00 | 3,00 | 3,00 | 3,00 | 3,00 | 3,00 | 3,00 | 3,00 |
| | Caprylic/Capric Triglyceride | 3,00 | 3,00 | 3,00 | 3,00 | 3,00 | 3,00 | 3,00 | 3,00 | 3,00 |
| | Octyldodecanol | 4,00 | 4,00 | 4,00 | 4,00 | 4,00 | 4,00 | 4,00 | 4,00 | 4,00 |
| | Dicaprylyl Ether | 3,50 | 3,50 | 3,50 | 3,50 | 3,50 | 3,50 | 3,50 | 3,50 | 3,50 |
| | Dicaprylyl Carbonate | 2,10 | 2,10 | 2,10 | 2,10 | 2,10 | 2,10 | 2,10 | 2,10 | 2,10 |
| | Glyceryl Stearate Citrate | 2,00 | 2,00 | 2,00 | 2,00 | 2,00 | 2,00 | 2,00 | 2,00 | 2,00 |
| | Diethylhexyl Butamido Triazine | 4,00 | 3,00 | 5,00 | 1,50 | 3,00 | 2,00 | 1,00 | 1,00 | 2,00 |

(continued)

| Example B3: Sun Milk | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Bis-Ethylhexyloxyphenol Methoxyphenyl Triazine | 1,00 | 2,00 | 3,00 | 2,50 | 3,00 | 2,00 | 1,00 | 3,00 | 1,00 |
| | Ethylhexyl Methoxycinnamate | 4,00 | 6,00 | 10,00 | 8,00 | 7,00 | 6,00 | 4,00 | 5,00 | 5,00 |
| | Titanium Dioxide | 7,00 | 8,00 | 2,00 | - | 1,50 | 1,00 | 3,00 | 1,00 | 2,00 |
| | Stearyl Alcohol | 1,50 | 1,50 | 1,50 | 1,50 | 1,50 | 1,50 | 1,50 | 1,50 | 1,50 |
| | Hydrogenated Coco-Glycerides | 1,40 | 1,40 | 1,40 | 1,40 | 1,40 | 1,40 | 1,40 | 1,40 | 1,40 |
| | PVP/Hexadecene Copolymer | 0,50 | 0,50 | 0,50 | 0,50 | 0,50 | 0,50 | 0,50 | 0,50 | 0,50 |
| Part B | Organic micro-particulate UV absorber of formula (3), (5) or (7) | 2,00 | 4,00 | 6,00 | 8,00 | 10,00 | 14,00 | 16,00 | 18,00 | 20,00 |
| Part C | Aqua | Qs to 100 | Qs to 100 | Qs to 100 | Qs to 100 | Qs to 100 | Qs to 100 | Qs to 100 | Qs to 100 | Qs to 100 |
| | Glycerin | 6,00 | 6,00 | 6,00 | 6,00 | 6,00 | 6,00 | 6,00 | 6,00 | 6,00 |
| | Xanthan Gum | 0,25 | 0,25 | 0,25 | 0,25 | 0,25 | 0,25 | 0,25 | 0,25 | 0,25 |
| | Acrylates/C10-30 Alkyl Acrylate Crosspolymer | 0,15 | 0,15 | 0,15 | 0,15 | 0,15 | 0,15 | 0,15 | 0,15 | 0,15 |
| | Trisodium EDTA | 0,20 | 0,20 | 0,20 | 0,20 | 0,20 | 0,20 | 0,20 | 0,20 | 0,20 |
| Part D | Alcohol Denatured | 4,00 | 4,00 | 4,00 | 4,00 | 4,00 | 4,00 | 4,00 | 4,00 | 4,00 |
| Part E | Sodium Hydroxide | Qs | Qs | Qs | Qs | Qs | Qs | Qs | Qs | Qs |
| | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Tocopheryl Acetate | 1,00 | 1,00 | 1,00 | 1,00 | 1,00 | 1,00 | 1,00 | 1,00 | 1,00 |
| In vitro SPF measured according the method described | | 37,4 | 49,6 | 52,1 | 28 | 41,7 | 30,9 | 26 | 29,3 | 30,3 |
| UVA PF measured according the method described | | 7,7 | 11,2 | 10,1 | 8,5 | 11,1 | 10,4 | 10,6 | 13 | 11 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then, part E is added to the emulsion formed and the pH is checked and adjusted with triethanolamine.

| Example B4: Water resistant Sunscreen Emulsion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Polyglyceryl-10 Pentastearate (and) Behenyl Alcohol (and) Sodium Stearoyl Lactylate | 2,50 | 2,50 | 2,50 | 2,50 | 2,50 | 2,50 | 2,50 | 2,50 | 2,50 |
| | VP/Eicosene Copolymer | 1,50 | 1,50 | 1,50 | 1,50 | 1,50 | 1,50 | 1,50 | 1,50 | 1,50 |
| | Stearyl Alcohol | 1,50 | 1,50 | 1,50 | 1,50 | 1,50 | 1,50 | 1,50 | 1,50 | 1,50 |
| | Squalane | 4,00 | 4,00 | 4,00 | 4,00 | 4,00 | 4,00 | 4,00 | 4,00 | 4,00 |
| | C12-15 Alkyl Benzoate | 5,50 | 5,50 | 5,50 | 5,50 | 5,50 | 5,50 | 5,50 | 5,50 | 5,50 |
| | Bis-Ethylhexyloxyphenol | 4,00 | 3,00 | 2,00 | 6,00 | 2,50 | 3,00 | 1,50 | 2,00 | 1,00 |
| | Methoxyphenyl Triazine | | | | | | | | | |
| | Ethylhexyl Methoxycinnamate | 4,00 | 6,00 | 5,00 | 8,00 | 4,00 | 3,00 | 2,00 | 1,50 | 1,00 |
| | Titanium Dioxide | 1,00 | 4,00 | 6,00 | 8,00 | 2,00 | 4,00 | 6,00 | 3,00 | 1,50 |
| | Octocrylene | 10,0 | 4,00 | 5,00 | 4,00 | 4,00 | 5,00 | 3,00 | 4,00 | 2,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Water | qs to 100 | qs to 100 | qs to 100 | qs to 100 | qs to 100 | qs to 100 | qs to 100 | qs to 100 | qs to 100 |
| | Glycerin | 1,80 | 1,80 | 1,80 | 1,80 | 1,80 | 1,80 | 1,80 | 1,80 | 1,80 |
| | Steareth-10 Allyl Ether/Acrylates Copolymer | 0,80 | 0,80 | 0,80 | 0,80 | 0,80 | 0,80 | 0,80 | 0,80 | 0,80 |
| | VP/Hexadecene Copolymer | 2,70 | 2,70 | 2,70 | 2,70 | 2,70 | 2,70 | 2,70 | 2,70 | 2,70 |
| Part D | Cyclomethicone | 1,50 | 1,50 | 1,50 | 1,50 | 1,50 | 1,50 | 1,50 | 1,50 | 1,50 |
| | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Aqua (and) Tocopheryl Acetate (and) Caprylic/Capric Triglyceride (and) Polysorbate 80 (and) Lecithin | 3,50 | 3,50 | 3,50 | 3,50 | 3,50 | 3,50 | 3,50 | 3,50 | 3,50 |
| | Fragrance | qs | qs | qs | qs | qs | qs | qs | qs | qs |
| | Water (and) Sodium Hydroxide | qs | qs | qs | qs | qs | qs | qs | qs | qs |
| | | | | | | | | | | |
| In vitro SPF measured according the method described | | 36 | 36,6 | 39,5 | 69,1 | 28 | 37,7 | 33,1 | 26,8 | 14 |
| UVA PF measured according the method described | | 9,7 | 11 | 11,6 | 20,6 | 11,1 | 14,7 | 13,7 | 13,2 | 10,8 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then the pH is checked and adjusted with triethanolamine.

Example B5: O/W Daily Care

| | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Cetearyl Alcohol (and) Dicetyl Phosphate (and) Ceteth-10 Phosphate | 1,50 | 1,50 | 1,50 | 1,50 | 1,50 | 1,50 | 1,50 | 1,50 | 1,50 |
| | C12-15 Alkyl Benzoate | 2,00 | 2,00 | 2,00 | 2,00 | 2,00 | 2,00 | 2,00 | 2,00 | 2,00 |
| | Dicaprylyl Ether | 3,00 | 3,00 | 3,00 | 3,00 | 3,00 | 3,00 | 3,00 | 3,00 | 3,00 |
| | Ethoxydiglycol Oleate | 2,00 | 2,00 | 2,00 | 2,00 | 2,00 | 2,00 | 2,00 | 2,00 | 2,00 |
| | Stearic Acid | 1,00 | 1,00 | 1,00 | 1,00 | 1,00 | 1,00 | 1,00 | 1,00 | 1,00 |
| | Sodium Acrylates Copolymer (and) Glycine Soja (and) PPG-1 Trideceth-6 | 2,00 | 2,00 | 2,00 | 2,00 | 2,00 | 2,00 | 2,00 | 2,00 | 2,00 |
| | Squalane | 3,50 | 3,50 | 3,50 | 3,50 | 3,50 | 3,50 | 3,50 | 3,50 | 3,50 |
| | Drometrizole trisiloxane | 10,00 | 8,00 | 7,00 | 5,00 | 4,00 | 3,00 | 2,00 | 1,50 | 1,00 |
| | Diethylamino hydroxybenzoyl Hexylbenzoate | 5,00 | 3,00 | 2,00 | 2,50 | 3,00 | 1,00 | 2,00 | 3,00 | 1,50 |
| | Ethylhexyl Triazone | 3,00 | 1,50 | 2,00 | 2,50 | 1,00 | 1,00 | 1,00 | 1,50 | 2,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | qs to 100 | qs to 100 | qs to 100 | qs to 100 | qs to 100 | qs to 100 | qs to 100 | qs to 100 | qs to 100 |
| Part D | Diazolidinyl Urea (and) Iodopropynyl Butylcarbamate | 0,15 | 0,15 | 0,15 | 0,15 | 0,15 | 0,15 | 0,15 | 0,15 | 0,15 |
| | Propylene Glycol | 2,50 | 2,50 | 2,50 | 2,50 | 2,50 | 2,50 | 2,50 | 2,50 | 2,50 |
| | Aqua | 10,0 | 10,0 | 10,0 | 10,0 | 10,0 | 10,0 | 10,0 | 10,0 | 10,0 |
| Part E | Cyclopentasiloxane, Dimethiconol | 2,00 | 2,00 | 2,00 | 2,00 | 2,00 | 2,00 | 2,00 | 2,00 | 2,00 |
| | Ethoxydiglycol | 5,00 | 5,00 | 5,00 | 5,00 | 5,00 | 5,00 | 5,00 | 5,00 | 5,00 |
| | Cyclopentasiloxane (and) Dimethicone/Vinyl-dimethicone Cross polymer | 2,00 | 2,00 | 2,00 | 2,00 | 2,00 | 2,00 | 2,00 | 2,00 | 2,00 |
| | Sodium Hydroxide | 0,10 | 0,10 | 0,10 | 0,10 | 0,10 | 0,10 | 0,10 | 0,10 | 0,10 |
| | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |

(continued)

| Example B5: O/W Daily Care | A | B | C | D | E | F | G | H | I |
|---|---|---|---|---|---|---|---|---|---|
| INCI-Name | % w/w | % w/w | % w/w | % w/w | % w/w | % w/w | % w/w | % w/w | % w/w |
| In vitro SPF measured according the method described | 29,6 | 16,5 | 17,4 | 18,4 | 11 | 9,6 | 9,5 | 12 | 13,1 |
| UVA PF measured according the method described | 15,8 | 11,8 | 10,4 | 11 | 11,9 | 9,3 | 10,9 | 12,8 | 10,6 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then, part E is added to the emulsion formed and the pH is checked and adjusted with sodium hydroxide.

| Example B6: Every Day Lotion | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Stearyl Phosphate | 5,00 | 5,00 | 5,00 | 5,00 | 5,00 | 5,00 | 5,00 | 5,00 | 5,00 |
| | Tricontanyl PVP | 1,00 | 1,00 | 1,00 | 1,00 | 1,00 | 1,00 | 1,00 | 1,00 | 1,00 |
| | Ethoxydiglycol Oleate | 3,00 | 3,00 | 3,00 | 3,00 | 3,00 | 3,00 | 3,00 | 3,00 | 3,00 |
| | Squalane | 5,00 | 5,00 | 5,00 | 5,00 | 5,00 | 5,00 | 5,00 | 5,00 | 5,00 |
| | C12-15 Alkyl Benzoate | 5,00 | 5,00 | 5,00 | 5,00 | 5,00 | 5,00 | 5,00 | 5,00 | 5,00 |
| | Glyceryl Stearate | 2,00 | 2,00 | 2,00 | 2,00 | 2,00 | 2,00 | 2,00 | 2,00 | 2,00 |
| | Cetyl Alcohol | 2,00 | 2,00 | 2,00 | 2,00 | 2,00 | 2,00 | 2,00 | 2,00 | 2,00 |
| | Diethylhexyl Butamido Triazone | 4,00 | 5,00 | 3,00 | 1,00 | 1,00 | 3,00 | 2,00 | 1,50 | 1,00 |
| | Ethylhexyl Methoxycinnamate | 6,00 | 8,00 | 4,00 | 3,00 | 2,00 | 4,00 | 5,00 | 3,00 | 3,00 |
| | Titanium Dioxide | 4,00 | 5,00 | 3,00 | 2,50 | 2,00 | 2,00 | 1,00 | 1,50 | 1,00 |
| Part B | Organic micro-particulate UV absorber of formula (3), (5) or (7) | 2,00 | 4,00 | 6,00 | 8,00 | 10,00 | 14,00 | 16,00 | 18,00 | 20,00 |
| Part C | Aqua | qs to 100 | qs to 100 | qs to 100 | qs to 100 | qs to 100 | qs to 100 | qs to 100 | qs to 100 | qs to 100 |
| | Steareth-10 Allyl Ether/ Acrylates Copolymer | 0,50 | 0,50 | 0,50 | 0,50 | 0,50 | 0,50 | 0,50 | 0,50 | 0,50 |
| | Glycerin | 2,50 | 2,50 | 2,50 | 2,50 | 2,50 | 2,50 | 2,50 | 2,50 | 2,50 |
| | Diazolidinyl Urea (and) Iodopropynyl Butylcarbamate | 0,15 | 0,15 | 0,15 | 0,15 | 0,15 | 0,15 | 0,15 | 0,15 | 0,15 |

(continued)

| Example B6: Every Day Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Disodium Phenyl Dibenzimidazole tetrasulfonate | 7,00 | 5,00 | 4,00 | 3,00 | 2,00 | 3,00 | 1,50 | 1,00 | 1,00 |
| | Sodium Lauroyl Glutamate | 0,70 | 0,70 | 0,70 | 0,70 | 0,70 | 0,70 | 0,70 | 0,70 | 0,70 |
| Part D | Cyclopentasiloxane (and) Dimethiconol | 1,50 | 1,50 | 1,50 | 1,50 | 1,50 | 1,50 | 1,50 | 1,50 | 1,50 |
| | Triethanolamine | 1,85 | 1,85 | 1,85 | 1,85 | 1,85 | 1,85 | 1,85 | 1,85 | 1,85 |
| | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |
| In vitro SPF measured according the method described | | 50,9 | 56,6 | 38,7 | 25,7 | 19,8 | 36,8 | 27,1 | 24,1 | 20,5 |
| UVA PF measured according the method described | | 9,9 | 10,8 | 10,7 | 10,7 | 10,2 | 13 | 10,9 | 11,5 | 11,7 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then the pH is checked and adjusted with triethanolamine.

| Example B7: Sun Cream | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Tribehenin PEG-20 esters | 5,00 | 5,00 | 5,00 | 5,00 | 5,00 | 5,00 | 5,00 | 5,00 | 5,00 |
| | Dibutyl adipate | 5,00 | 5,00 | 5,00 | 5,00 | 5,00 | 5,00 | 5,00 | 5,00 | 5,00 |
| | PPG-2 Myristyl Ether Propionate | 5,00 | 5,00 | 5,00 | 5,00 | 5,00 | 5,00 | 5,00 | 5,00 | 5,00 |
| | Octocrylene | 10,00 | 4,00 | 5,00 | 4,00 | 4,00 | 5,00 | 3,00 | 4,00 | 2,00 |
| | Ethylhexyl Triazone | 3,00 | 1,50 | 2,00 | 2,50 | 1,00 | 1,00 | 1,00 | 1,50 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Ethylhexyl Methoxycinnamate | 6,00 | 8,00 | 4,00 | 3,00 | 2,00 | 4,00 | 5,00 | 3,00 | 3,00 |
| | Butyl Methoxy Dibenzoyl Methane | 3,00 | 2,00 | 2,50 | 3,00 | 2,00 | 1,50 | 1,50 | 1,00 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |

(continued)

| Example B7: Sun Cream | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part C | Water | Qs to 100 | Qsto 100 | Qsto 100 | Qsto 100 | Qsto 100 | Qsto 100 | Qsto 100 | Qsto 100 | Qsto 100 |
| | PVP/dimethylconylacrylate/ poly carbamyl/polyglycol ester | 3,00 | 3,00 | 3,00 | 3,00 | 3,00 | 3,00 | 3,00 | 3,00 | 3,00 |
| | Disodium EDTA | 0,20 | 0,20 | 0,20 | 0,20 | 0,20 | 0,20 | 0,20 | 0,20 | 0,20 |
| | Sclerotium Gum | 2,00 | 2,00 | 2,00 | 2,00 | 2,00 | 2,00 | 2,00 | 2,00 | 2,00 |
| | Ammonium Acryldimethyltaurate/Beneth-25 Methacrylate Crosspolymer. | 0,50 | 0,50 | 0,50 | 0,50 | 0,50 | 0,50 | 0,50 | 0,50 | 0,50 |
| Part D | Cyclopentasiloxane (and) cyclohexasiloxane | 4,00 | 4,00 | 4,00 | 4,00 | 4,00 | 4,00 | 4,00 | 4,00 | 4,00 |
| | Aqua (and) Tocopheryl Acetate (and) Caprylic/Capric Triglyceride (and) Polysorbate 80 (and) Lecithin | 0,50 | 0,50 | 0,50 | 0,50 | 0,50 | 0,50 | 0,50 | 0,50 | 0,50 |
| | Phenoxyethanol (and) Methylparaben (and) Ethylparaben (and) Butylparaben (and) Propylparaben (and) Isobutylparaben | 1,00 | 1,00 | 1,00 | 1,00 | 1,00 | 1,00 | 1,00 | 1,00 | 1,00 |
| | Sodium Hydroxide (and) Water | qs | qs | qs | qs | qs | qs | qs | qs | qs |
| In vitro SPF measured according the method described | | 54,3 | 34,4 | 30,9 | 53,2 | 24,1 | 29,3 | 25,2 | 29,2 | 24,3 |
| UVA PF measured according the method described | | 12,6 | 9,8 | 10,1 | 17,7 | 12,6 | 14,6 | 10,9 | 12,8 | 10,9 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added. Then the pH is checked and adjusted with sodium hydroxide

| Example B8: Daily Care Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Polyglyceryl Methyl Glucose Distearate | 2,50 | 2,50 | 2,50 | 2,50 | 2,50 | 2,50 | 2,50 | 2,50 | 2,50 |
| | Cetearyl Alcohol | 2,00 | 2,00 | 2,00 | 2,00 | 2,00 | 2,00 | 2,00 | 2,00 | 2,00 |
| | Octyl Stearate | 3,00 | 3,00 | 3,00 | 3,00 | 3,00 | 3,00 | 3,00 | 3,00 | 3,00 |

(continued)

| Example B8: Daily Care Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Caprylic/Capric Triglyceride | 4,00 | 4,00 | 4,00 | 4,00 | 4,00 | 4,00 | 4,00 | 4,00 | 4,00 |
| | Isohexadecane | 4,00 | 4,00 | 4,00 | 4,00 | 4,00 | 4,00 | 4,00 | 4,00 | 4,00 |
| | Polysilicone-15 | 8,00 | 6,00 | 4,00 | 3,50 | 2,50 | 2,00 | 1,50 | 1,00 | 1,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Ethylhexyl Triazone | 3,00 | 1,50 | 2,00 | 2,50 | 1,00 | 1,00 | 1,00 | 1,50 | 2,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | qs to 100 | qs to 100 | qs to 100 | qs to 100 | qs to 100 | qs to 100 | qs to 100 | qs to 100 | qs to 100 |
| | Terephthalidene Dicamphorsulfonic acid | 8,00 | 6,00 | 4,00 | 3,00 | 2,00 | 2,00 | 1,00 | 1,50 | 1,00 |
| | Glycerin | 5,00 | 5,00 | 5,00 | 5,00 | 5,00 | 5,00 | 5,00 | 5,00 | 5,00 |
| Part D | Phenoxyethanol (and) Methylparaben (and) Butylparaben (and) Ethylparaben (and) Propylparaben | 0,50 | 0,50 | 0,50 | 0,50 | 0,50 | 0,50 | 0,50 | 0,50 | 0,50 |
| | Cyclomethicone (and) Dimethicone | 3,00 | 3,00 | 3,00 | 3,00 | 3,00 | 3,00 | 3,00 | 3,00 | 3,00 |
| | Steareth-10 Allyl Ether/ Acrylates Copolymer | 0,50 | 0,50 | 0,50 | 0,50 | 0,50 | 0,50 | 0,50 | 0,50 | 0,50 |
| In vitro SPF measured according the method described | | 44,3 | 34,5 | 33,7 | 57,1 | 35,2 | 49 | 47,4 | 56,9 | 57,3 |
| UVA PF measured according the method described | | 22 | 18 | 13,9 | 21,5 | 13,6 | 16,3 | 14 | 15 | 12,8 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added.

| Example B9: O/W Every Day UV Protection Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Glyceryl Stearate (and) PEG-100 Stearate | 5,00 | 5,00 | 5,00 | 5,00 | 5,00 | 5,00 | 5,00 | 5,00 | 5,00 |
| | Stearyl Alcohol | 1,00 | 1,00 | 1,00 | 1,00 | 1,00 | 1,00 | 1,00 | 1,00 | 1,00 |
| | Tripalmitin | 0,70 | 0,70 | 0,70 | 0,70 | 0,70 | 0,70 | 0,70 | 0,70 | 0,70 |

(continued)

| Example B9: O/W Every Day UV Protection Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Dimethicone | 2,00 | 2,00 | 2,00 | 2,00 | 2,00 | 2,00 | 2,00 | 2,00 | 2,00 |
| | C12-15 Alkyl Benzoate | 5,00 | 5,00 | 5,00 | 5,00 | 5,00 | 5,00 | 5,00 | 5,00 | 5,00 |
| | Isopropyl Palmitate | 5,00 | 5,00 | 5,00 | 5,00 | 5,00 | 5,00 | 5,00 | 5,00 | 5,00 |
| | Drometrizole trisiloxane | 10,00 | 8,00 | 7,00 | 5,00 | 4,00 | 3,00 | 2,00 | 1,50 | 1,00 |
| | Ethylhexyl Methoxycinnamate | 6,00 | 8,00 | 4,00 | 3,00 | 2,00 | 4,00 | 5,00 | 3,00 | 3,00 |
| | Titanium dioxide | 4,00 | 5,00 | 3,00 | 2,50 | 2,00 | 2,00 | 1,00 | 1,50 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Water | qs to 100 | qs to 100 | qs to 100 | qs to 100 | qs to 100 | qs to 100 | qs to 100 | qs to 100 | qs to 100 |
| | Polysorbate 60 | 0,50 | 0,50 | 0,50 | 0,50 | 0,50 | 0,50 | 0,50 | 0,50 | 0,50 |
| | Glycerin | 3,00 | 3,00 | 3,00 | 3,00 | 3,00 | 3,00 | 3,00 | 3,00 | 3,00 |
| Part D | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Steareth-10 Allyl Ether/ Acrylates Copolymer | 1,50 | 1,50 | 1,50 | 1,50 | 1,50 | 1,50 | 1,50 | 1,50 | 1,50 |
| Part E | Water (and) Sodium Hydroxide | qs | qs | qs | qs | qs | qs | qs | qs | qs |
| | Disodium Phenyl Dibenzimidazole Tetrasulfonate | 7,00 | 5,00 | 4,00 | 3,00 | 2,00 | 3,00 | 1,50 | 1,00 | 1,00 |
| Part F | Fragrance | qs | qs | qs | qs | qs | qs | qs | qs | qs |
| In vitro SPF measured according the method described | | 47,6 | 50,2 | 41,6 | 38,3 | 36 | 49,1 | 45,9 | 47,8 | 51,9 |
| UVA PF measured according the method described | | 15,8 | 14,4 | 12,9 | 11,4 | 10,5 | 12,1 | 10,6 | 10,7 | 11,3 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part E and D are added . Then, incorporate fragrance and check the pH.

| Example B10: O/W Sprayable Lotion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Cetearyl Alcohol (and) Polysorbate Oil | 3,50 | 3,50 | 3,50 | 3,50 | 3,50 | 3,50 | 3,50 | 3,50 | 3,50 |
| | PEG-40 Hydrogenated Castor Oil | 4,00 | 4,00 | 4,00 | 4,00 | 4,00 | 4,00 | 4,00 | 4,00 | 4,00 |
| | Almond oil seet (prunus dulcis) | 1,00 | 1,00 | 1,00 | 1,00 | 1,00 | 1,00 | 1,00 | 1,00 | 1,00 |
| | Glyceryl Stearate | 1,00 | 1,00 | 1,00 | 1,00 | 1,00 | 1,00 | 1,00 | 1,00 | 1,00 |
| | PEG-7 Glyceryl Cocoate | 2,00 | 2,00 | 2,00 | 2,00 | 2,00 | 2,00 | 2,00 | 2,00 | 2,00 |
| | Isopropyl Palmitate | 4,00 | 4,00 | 4,00 | 4,00 | 4,00 | 4,00 | 4,00 | 4,00 | 4,00 |
| | Diethylhexyl Butamido Triazone | 3,00 | 1,50 | 2,00 | 2,50 | 1,00 | 1,00 | 1,00 | 1,50 | 2,00 |
| | Octocrylene | 10,0 | 4,00 | 5,00 | 4,00 | 4,00 | 5,00 | 3,00 | 4,00 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Diethylamino Hydroxybenzoyl hexyl benzoate | 5,00 | 3,00 | 2,00 | 2,50 | 3,00 | 1,00 | 2,00 | 3,00 | 1,50 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Water | Qs to 100 | Qs to 100 | Qs to 100 | Qs to 100 | Qs to 100 | Qs to 100 | Qs to 100 | Qs to 100 | Qs to 100 |
| | Glycerine | 2,00 | 2,00 | 2,00 | 2,00 | 2,00 | 2,00 | 2,00 | 2,00 | 2,00 |
| | Propylene glycol | 2,50 | 2,50 | 2,50 | 2,50 | 2,50 | 2,50 | 2,50 | 2,50 | 2,50 |
| | Terephthalidene Dicamphor sulfonic acid | 8,00 | 6,00 | 4,00 | 3,00 | 2,00 | 2,00 | 1,00 | 1,50 | 1,00 |
| Part D | Preservative (Nipa) | q.s. | q.s. | q.s. | q.s. | q.s. | q.s. | q.s. | q.s. | q.s. |
| | Perfume Oil (Luzi) | q.s. | q.s. | q.s. | q.s. | q.s. | q.s. | q.s. | q.s. | q.s. |
| In vitro SPF measured according the method described | | 73,6 | 39,2 | 31,9 | 54,8 | 25,7 | 28,5 | 18,1 | 28,4 | 21,7 |
| UVA PF measured according the method described | | 38 | 27,5 | 20,8 | 28,2 | 20,8 | 19,2 | 16 | 21,2 | 15,1 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Below 65°C the ingredients of part B are added separately. After cooling down under moderate stirring to 40°C part D is added.

| Example B11: Cold Process Spray | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Sorbitan-30 | 3,00 | 3,00 | 3,00 | 3,00 | 3,00 | 3,00 | 3,00 | 3,00 | 3,00 |

(continued)

| Example B11: Cold Process Spray | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part B | Isostearyl Alcohol | 6,00 | 6,00 | 6,00 | 6,00 | 6,00 | 6,00 | 6,00 | 6,00 | 6,00 |
| | Sorbitan Oleate (and) Polyglycerol 3-Polyricinoleate | 2,00 | 2,00 | 2,00 | 2,00 | 2,00 | 2,00 | 2,00 | 2,00 | 2,00 |
| | Polysorbate-20 | 1,00 | 1,00 | 1,00 | 1,00 | 1,00 | 1,00 | 1,00 | 1,00 | 1,00 |
| | Dimethicone | 1,00 | 1,00 | 1,00 | 1,00 | 1,00 | 1,00 | 1,00 | 1,00 | 1,00 |
| | Octocrylene | 10,0 0 | 4,00 | 5,00 | 4,00 | 4,00 | 5,00 | 3,00 | 4,00 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Titanium dioxide | 4,00 | 5,00 | 3,00 | 2,50 | 2,00 | 2,00 | 1,00 | 1,50 | 1,00 |
| Part C | Organic micro-particulate UV absorber of formula (3), (5) or (7) | 2,00 | 4,00 | 6,00 | 8,00 | 10,0 | 14,0 | 16,0 | 18,0 | 20,0 |
| Part D | DMDM Hydantoin | 1,00 | 1,00 | 1,00 | 1,00 | 1,00 | 1,00 | 1,00 | 1,00 | 1,00 |
| | Aqua | Qsto 100 | Qsto 100 | Qsto 100 | Qsto 100 | Qsto 100 | Qsto 100 | Qsto 100 | Qsto 100 | Qsto 100 |
| | Hydroxypropyl Starch Phosphate | 4,00 | 4,00 | 4,00 | 4,00 | 4,00 | 4,00 | 4,00 | 4,00 | 4,00 |
| | Acrylates Copolymer | 3,35 | 3,35 | 3,35 | 3,35 | 3,35 | 3,35 | 3,35 | 3,35 | 3,35 |
| | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |
| | | | | | | | | | | |
| In vitro SPF measured according the method described | | 35,4 | 27,1 | 19,1 | 31,9 | 17,8 | 22,3 | 11,8 | 16,5 | 10,2 |
| UVA PF measured according the method described | | 11,9 | 11,5 | 9,6 | 16,3 | 10,9 | 13,1 | 10,3 | 12,1 | 10,3 |

Manufacturing instruction:

Part A is prepared by incorporating all ingredients, then stirred under moderate speed. Part D is prepared. At room temperature, part A is poured into part D under progressive stirring speed. Below 65°C the ingredients of part C are added separately. Then the pH is checked and adjusted.

| Example B12: Sun spray foaming | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Behenyl Alcohol (and) Glyceryl Stearate (and) Glyceryl Stearate Citrate (and) Disodium Ethylene Di(Cocamide PEG-15 Disulfate) | 0,50 | 0,50 | 0,50 | 0,50 | 0,50 | 0,50 | 0,50 | 0,50 | 0,50 |

(continued)

| Example B12: Sun spray foaming | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Isotrideceth-12 | 0,50 | 0,50 | 0,50 | 0,50 | 0,50 | 0,50 | 0,50 | 0,50 | 0,50 |
| | Ethylhexyl Salicylate | 4,00 | 4,00 | 4,00 | 4,00 | 4,00 | 4,00 | 4,00 | 4,00 | 4,00 |
| | Hydrogenated Coco-glycerides | 1,50 | 1,50 | 1,50 | 1,50 | 1,50 | 1,50 | 1,50 | 1,50 | 1,50 |
| | C12-15 Alkyl Benzoate | 8,00 | 8,00 | 8,00 | 8,00 | 8,00 | 8,00 | 8,00 | 8,00 | 8,00 |
| | Ditehylhexyl butamido Triazone | 3,00 | 1,50 | 2,00 | 2,50 | 1,00 | 1,00 | 1,00 | 1,50 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Octocrylene | 10,0 | 4,00 | 5,00 | 4,00 | 4,00 | 5,00 | 3,00 | 4,00 | 2,00 |
| | Butyl Methoxy Dibenzoyl Methane | 3,00 | 2,00 | 2,50 | 3,00 | 2,00 | 1,50 | 1,50 | 1,00 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | 5,00 | 5,00 | 5,00 | 5,00 | 5,00 | 5,00 | 5,00 | 5,00 | 5,00 |
| | Glycerin | 4,00 | 4,00 | 4,00 | 4,00 | 4,00 | 4,00 | 4,00 | 4,00 | 4,00 |
| | Galactoarabinan | 0,25 | 0,25 | 0,25 | 0,25 | 0,25 | 0,25 | 0,25 | 0,25 | 0,25 |
| Part D | Disodium Ethylene Di(Cocamide PEG-15 Disulfate) (and) Sodium Lauroyl Lactylate | 1,00 | 1,00 | 1,00 | 1,00 | 1,00 | 1,00 | 1,00 | 1,00 | 1,00 |
| | Water | Qs to 100 | Qs to 100 | Qs to 100 | Qs to 100 | Qs to 100 | Qs to 100 | Qs to 100 | Qs to 100 | Qs to 100 |
| | Zinc Oxide | 4,00 | 5,00 | 3,00 | 2,50 | 2,00 | 2,00 | 1,00 | 1,50 | 1,00 |
| Part E | Phenoxyethanol (and) Methylparaben (and) Ethylparaben (and) Butylparaben (and) Propylparaben (and) Isobutylparaben | 0,70 | 0,70 | 0,70 | 0,70 | 0,70 | 0,70 | 0,70 | 0,70 | 0,70 |
| | Tocopheryl Acetate | 0,60 | 0,60 | 0,60 | 0,60 | 0,60 | 0,60 | 0,60 | 0,60 | 0,60 |
| In vitro SPF measured according the method described | | 58,8 | 27,6 | 28,2 | 53,7 | 21,4 | 25,7 | 16,4 | 23,1 | 19,3 |
| UVA PF measured according the method described | | 19,4 | 14,9 | 13,4 | 22,8 | 14,3 | 16,2 | 12,6 | 14 | 11,8 |

Manufacturing instruction:

Part A is prepared by incorporating all ingredients, then stirred under moderate speed and heated to 75°C. Part C is prepared and heated to 75°C. At this temperature, part A is poured into part C under progressive stirring speed. Immediately after homogenization of the mixture, part D is poured under stirring. Below 65°C the ingredients of part B are added separately. After cooling down under moderate stirring to 40°C part E is added . Then, check the pH.

| Example B13:O/W Sunscreen Cream | | | | | | | | | | |
|---|---|---|---|---|---|---|---|---|---|---|
| Foameous | | A | B | C | D | E | F | G | H | I |
| | INCI-Name | % w/w | % w/w | % w/w | % w/w | % w/w | % w/w | % w/w | % w/w | % w/w |
| Part A | Stearic Acid | 1,00 | 1,00 | 1,00 | 1,00 | 1,00 | 1,00 | 1,00 | 1,00 | 1,00 |
| | Cetearyl Alcohol | 2,00 | 2,00 | 2,00 | 2,00 | 2,00 | 2,00 | 2,00 | 2,00 | 2,00 |
| | PEG-20-Stearate | 1,00 | 1,00 | 1,00 | 1,00 | 1,00 | 1,00 | 1,00 | 1,00 | 1,00 |
| | Caprylic Acid/Caprinic Acid Triglyceride | 2,00 | 2,00 | 2,00 | 2,00 | 2,00 | 2,00 | 2,00 | 2,00 | 2,00 |
| | Paraffin Oil | 10,00 | 10,00 | 10,00 | 10,00 | 10,00 | 10,00 | 10,00 | 10,00 | 10,00 |
| | Cyclomethicone | 5,0 | 5,0 | 5,0 | 5,0 | 5,0 | 5,0 | 5,0 | 5,0 | 5,0 |
| | Dimethicone | 0,5 | 0,5 | 0,5 | 0,5 | 0,5 | 0,5 | 0,5 | 0,5 | 0, |
| | Drometrizole Trisiloxane | 10,0 | 8,00 | 7,00 | 5,00 | 4,00 | 3,00 | 2,00 | 1,50 | 1,00 |
| | Polysilicone-15 | 6,0 | 8,0 | 4,0 | 3,0 | 2,0 | 4,0 | 5,0 | 3, | 3,0 |
| | Bis-Ethylhexyloxyphenol Methoxyphenyl Triazine | 4,0 | 3,0 | 2,0 | 6,0 | 2,5 | 3,0 | 1,5 | 2,0 | 1,0 |
| | Dimethicone /Vinyl Dimethicone Crosspolymer | 1,5 | 1,5 | 1,5 | 1,5 | 1,5 | 1,5 | 1,5 | 1,5 | 1,5 |
| | Octylisostearate | 2,00 | 2,00 | 2,00 | 2,00 | 2,00 | 2,00 | 2,00 | 2,00 | 2,00 |
| | Myristyl Myristate | 1,0 | 1,0 | 1,0 | 1,0 | 1,0 | 1,0 | 1,0 | 1,0 | 1,0 |
| Part B | Glycerine | 6,0 | 6,0 | 6,0 | 6,0 | 6,0 | 6,0 | 6,0 | 6,0 | 6,0 |
| | Carboxymethylcellulose | 0,05 | 0,05 | 0,05 | 0,05 | 0,05 | 0,05 | 0,05 | 0,05 | 0,05 |
| | Magnesium Aluminium Silicate | 0,50 | 0,50 | 0,50 | 0,50 | 0,50 | 0,50 | 0,50 | 0,50 | 0,50 |
| | PEG-180/Laureth-50/TMMG Copolymer | 0,50 | 0,50 | 0,50 | 0,50 | 0,50 | 0,50 | 0,50 | 0,50 | 0,50 |
| | Talcum | 0,50 | 0,50 | 0,50 | 0,50 | 0,50 | 0,50 | 0,50 | 0,50 | 0,50 |
| | BHT | 0,02 | 0,02 | 0,02 | 0,02 | 0,02 | 0,02 | 0,02 | 0,02 | 0,02 |
| | Phenylbenzimidazole sulfonic acid | 7,0 | 5,0 | 4,0 | 3,0 | 2,0 | 3,0 | 1,5 | 1,0 | 1,0 |
| | Disodium EDTA | 0,10 | 0,10 | 0,10 | 0,10 | 0,10 | 0,10 | 0,10 | 0,10 | 0,10 |
| | KOH | q.s | q.s | q.s | q.s | q.s | q.s | q.s | q.s | q.s |
| | Water | Qs to 100 | Qs to 100 | Qs to 100 | Qs to 100 | Qs to 100 | Qs to 100 | Qs to 100 | Qs to 100 | Qs to 100 |
| Part C | Organic micro-particulate UV absorber of formula (3), (5) or (7) | 2,0 | 4,0 | 6,0 | 8,0 | 10, | 14,0 | 16,0 | 18,0 | 20,0 |
| Part E | Parfum, Preservative, Dyes | q.s. | q.s. | q.s. | q.s. | q.s. | q.s. | q.s. | q.s. | q.s. |
| In vitro SPF measured according the method described | | 55,9 | 45,8 | 35,4 | 43,5 | 23,2 | 31,8 | 18 | 15,5 | 12,5 |
| UVA PF measured according the method described | | 12,8 | 11,4 | 10,2 | 16,5 | 11 | 12,6 | 10,3 | 11,3 | 9,9 |

Manufacturing instruction:

Part A is prepared by incorporating all ingredients, then stirred under moderate speed and heated to 75°C. Part B is prepared and heated to 75°C. At this temperature, part A is poured into part B under progressive stirring speed. Below 65°C the ingredient of part C is added separately. After cooling down under moderate stirring to 40°C part E is added . Then, check the pH.

Example B14:Continuous Spray Fresh Cooling

| | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | SD-Alcohol 40 | 58,00 | 58,00 | 58,00 | 58,00 | 58,00 | 58,00 | 58,00 | 58,00 | 58,00 |
| | Diethylhexyl 2,6-Naphthalate | 6,00 | 6,00 | 6,00 | 6,00 | 6,00 | 6,00 | 6,00 | 6,00 | 6,00 |
| | Trisiloxane (and) Dimethicone | 4,00 | 4,00 | 4,00 | 4,00 | 4,00 | 4,00 | 4,00 | 4,00 | 4,00 |
| | Acrylates/Octylacrylamide Copolymer | 3,00 | 3,00 | 3,00 | 3,00 | 3,00 | 3,00 | 3,00 | 3,00 | 3,00 |
| | PPG-5-Ceteth-20 | 2,00 | 2,00 | 2,00 | 2,00 | 2,00 | 2,00 | 2,00 | 2,00 | 2,00 |
| | Tocopheryl Acetate | 1,00 | 1,00 | 1,00 | 1,00 | 1,00 | 1,00 | 1,00 | 1,00 | 1,00 |
| | Ascorbyl Palmitate | 0,3 | 0,3 | 0,3 | 0,3 | 0,3 | 0,3 | 0,3 | 0,3 | 0,3 |
| | Retinyl Palmitate | 0,3 | 0,3 | 0,3 | 0,3 | 0,3 | 0,3 | 0,3 | 0,3 | 0,3 |
| | Cyclopentasiloxane (and) Acrylates/Dimethicone Copolymer | 1,00 | 1,00 | 1,00 | 1,00 | 1,00 | 1,00 | 1,00 | 1,00 | 1,00 |
| | Diethylamino Hydroxybenzoyl hexyl Benzoate | 5,00 | 3,00 | 2,00 | 2,50 | 3,00 | 1,00 | 2,00 | 3,00 | 1,50 |
| | Ethylhexyl triazone | 3,00 | 1,50 | 2,00 | 2,50 | 1,00 | 1,00 | 1,00 | 1,50 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Propylene Glycol (and) Ethoxydiglycol (and) Menthyl PCA (and) Lauryl PCA | 1,50 | 1,50 | 1,50 | 1,50 | 1,50 | 1,50 | 1,50 | 1,50 | 1,50 |
| Part C | Organic micro-particulate UV absorber of formula (3), (5) or (7) | 2,00 | 4,00 | 6,00 | 8,00 | 10,00 | 14,00 | 16,00 | 18,00 | 20,00 |
| Part D | Phenylbenzimidazole sulfonic acid | 7,00 | 5,00 | 4,00 | 3,00 | 2,00 | 3,00 | 1,50 | 1,00 | 1,00 |
| | Parfume | q.s. | q.s. | q.s. | q.s. | q.s. | q.s. | q.s. | q.s. | q.s. |
| | Aqua | Qs to 100 | Qs to 100 | Qs to 100 | Qs to 100 | Qs to 100 | Qs to 100 | Qs to 100 | Qs to 100 | Qs to 100 |
| | | | | | | | | | | |
| Part B | Propellent | Qs | Qs | Qs | Qs | Qs | Qs | Qs | Qs | Qs |
| In vitro SPF measured according the method described | | 60,5 | 40,3 | 34,9 | 51,4 | 24,8 | 31,1 | 19,1 | 22,4 | 20,8 |
| UVA PF measured according the method described | | 17,1 | 12,4 | 9,9 | 18,6 | 13,9 | 12,6 | 12,3 | 15,5 | 11,7 |

Manufacturing instruction:

Part A is prepared by incorporating all ingredients, then stirred under moderate speed .Part D is prepared. Part A is poured into part D under progressive stirring speed. Immediately after homogenization of the mixture, part C is poured under stirring. Part B is added to tne mixture by specific process for continuous spray system.

| Example B15: PEG-free sunscreen | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Hexyldecanol | 2,30 | 2,30 | 2,30 | 2,30 | 2,30 | 2,30 | 2,30 | 2,30 | 2,30 |
| | Polyglyceryl-3 Methylglucose Distearate | 1,40 | 1,40 | 1,40 | 1,40 | 1,40 | 1,40 | 1,40 | 1,40 | 1,40 |
| | Polyglyceryl polyhydroxy stearate | 1,40 | 1,40 | 1,40 | 1,40 | 1,40 | 1,40 | 1,40 | 1,40 | 1,40 |
| | Cetyl Ethylhexanoate | 3,00 | 3,00 | 3,00 | 3,00 | 3,00 | 3,00 | 3,00 | 3,00 | 3,00 |
| | Isohexadecane | 3,00 | 3,00 | 3,00 | 3,00 | 3,00 | 3,00 | 3,00 | 3,00 | 3,00 |
| | Octocrylene | 10,0 | 4,00 | 5,00 | 4,00 | 4,00 | 5,00 | 3,00 | 4,00 | 2,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Ethylhexyl Methoxycinnamate | 6,00 | 8,00 | 4,00 | 3,00 | 2,00 | 4,00 | 5,00 | 3,00 | 3,00 |
| | Butyl MethoxyDibenzoylMethane | 3,00 | 2,00 | 2,50 | 3,00 | 2,00 | 1,50 | 1,50 | 1,00 | 1,00 |
| | Titanium Dioxide | 4,00 | 5,00 | 3,00 | 2,50 | 2,00 | 2,00 | 1,00 | 1,50 | 1,00 |
| | Phenoxyethanol (and) Methylparaben (and) Ethylparaben (and) Butylparaben (and) Propylparaben (and) Isobutylparaben | 0,60 | 0,60 | 0,60 | 0,60 | 0,60 | 0,60 | 0,60 | 0,60 | 0,60 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Water | Qsto 100 | Qsto 100 | Qsto 100 | Qsto 100 | Qsto 100 | Qsto 100 | Qsto 100 | Qsto 100 | Qsto 100 |
| | Phenylbenzimidazole sulfonic acid | 7,00 | 5,00 | 4,00 | 3,00 | 2,00 | 3,00 | 1,50 | 1,00 | 1,00 |
| | Glycerin | 3,00 | 3,00 | 3,00 | 3,00 | 3,00 | 3,00 | 3,00 | 3,00 | 3,00 |
| Part D | Sodium Acrylates Copolymer (and) Mineral Oil (and) PPG-1 Trideceth-6 | 1,50 | 1,50 | 1,50 | 1,50 | 1,50 | 1,50 | 1,50 | 1,50 | 1,50 |
| | Cyclopentasiloxane | 1,50 | 1,50 | 1,50 | 1,50 | 1,50 | 1,50 | 1,50 | 1,50 | 1,50 |
| In vitro SPF measured according the method described | | 74,6 | 58,3 | 45,2 | 56,9 | 35,3 | 44 | 30,8 | 33,9 | 23,5 |
| UVA PF measured according the method described | | 15,3 | 13,1 | 11,4 | 19,1 | 13,5 | 15,2 | 11,6 | 13,8 | 11,5 |

Manufacturing instruction:

Part A is prepared by incorporating all ingredients, then stirred under moderate speed. Part C is prepared. At room temperature, part A is poured into part C under progressive stirring speed. Part D is incorporated into the previously obtained mixture, under moderate stirring. Then the ingredient of part B is added separately. Then the pH is checked and adjusted.

| Example B16: Skin Protection Sunscreen W/O | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Glyceryl Oleate | 4,50 | 4,50 | 4,50 | 4,50 | 4,50 | 4,50 | 4,50 | 4,50 | 4,50 |
| | PEG-7 Hydrogenated Castor Oil | 1,50 | 1,50 | 1,50 | 1,50 | 1,50 | 1,50 | 1,50 | 1,50 | 1,50 |
| | Hydrogenated Castor Oil | 0,50 | 0,50 | 0,50 | 0,50 | 0,50 | 0,50 | 0,50 | 0,50 | 0,50 |
| | Microcrystalline Wax | 2,00 | 2,00 | 2,00 | 2,00 | 2,00 | 2,00 | 2,00 | 2,00 | 2,00 |
| | Beeswax | 1,50 | 1,50 | 1,50 | 1,50 | 1,50 | 1,50 | 1,50 | 1,50 | 1,50 |
| | C12-15 Alkyl Benzoate | 3,00 | 3,00 | 3,00 | 3,00 | 3,00 | 3,00 | 3,00 | 3,00 | 3,00 |
| | Isopropyl Isostearate | 2,50 | 2,50 | 2,50 | 2,50 | 2,50 | 2,50 | 2,50 | 2,50 | 2,50 |
| | Mineral Oil | 5,00 | 5,00 | 5,00 | 5,00 | 5,00 | 5,00 | 5,00 | 5,00 | 5,00 |
| | Diethylamino hydroxybenzoyl hexyl Benzoate | 5,00 | 3,00 | 2,00 | 2,50 | 3,00 | 1,00 | 2,00 | 3,00 | 1,50 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Ethylhexyl Methoxycinnamate | 6,00 | 8,00 | 4,00 | 3,00 | 2,00 | 4,00 | 5,00 | 3,00 | 3,00 |
| | Ethylhexyl triazone | 3,00 | 1,50 | 2,00 | 2,50 | 1,00 | 1,00 | 1,00 | 1,50 | 2,00 |
| | Drometrizole trisiloxane | 10,00 | 8,00 | 7,00 | 5,00 | 4,00 | 3,00 | 2,00 | 1,50 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | qs to 100 | qs to 100 | qs to 100 | qs to 100 | qs to 100 | qs to 100 | qs to 100 | qs to 100 | qs to 100 |
| | Magnesium Sulfate | 0,50 | 0,50 | 0,50 | 0,50 | 0,50 | 0,50 | 0,50 | 0,50 | 0,50 |
| | Terephthalidene dicamphor sulfonic acid | 8,00 | 6,00 | 4,00 | 3,00 | 2,00 | 2,00 | 1,00 | 1,50 | 1,00 |
| Part D | Citric Acid | 0,05 | 0,05 | 0,05 | 0,05 | 0,05 | 0,05 | 0,05 | 0,05 | 0,05 |
| | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |
| In vitro SPF measured according the method described | | 82,3 | 59,6 | 45,5 | 64,6 | 29,9 | 33,8 | 26,9 | 29,5 | 24,7 |
| UVA PF measured according the method described | | 43,9 | 32,6 | 24,7 | 30,9 | 22,7 | 20,4 | 16,9 | 20,7 | 14,5 |

Manufacturing instruction:

Part A is prepared by incorporating all ingredients, then stirred under moderate speed and heated to 75°C. Part C is prepared and heated to 75°C. At this temperature, part C is poured into part A under progressive stirring speed. Below 65°C the ingredient of part B is added separately. After cooling down under moderate stirring to 40°C part D is added . Then, check the pH.

| Example B17: W/Si sun cream | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Cyclopentasiloxane (and) PEG/PPG-18/18 Dimethicone | 10,0 | 10,0 | 10,0 | 10,0 | 10,0 | 10,0 | 10,0 | 10,0 | 10,0 |
| | Cyclomethicone | 9,00 | 9,00 | 9,00 | 9,00 | 9,00 | 9,00 | 9,00 | 9,00 | 9,00 |
| | Cyclomethicone (and) Trimethylsiloxysilicate | 3,50 | 3,50 | 3,50 | 3,50 | 3,50 | 3,50 | 3,50 | 3,50 | 3,50 |
| | Cyclopentasiloxane (and) Dimethicone Crosspolymer | 7,00 | 7,00 | 7,00 | 7,00 | 7,00 | 7,00 | 7,00 | 7,00 | 7,00 |
| | Polysilicone-15 | 6,00 | 8,00 | 4,00 | 3,00 | 2,00 | 4,00 | 5,00 | 3,00 | 3,00 |
| | Diethylhexyl Butamido Triazone | 3,00 | 1,50 | 2,00 | 2,50 | 1,00 | 1,00 | 1,00 | 1,50 | 2,00 |
| | Titanium Dioxide | 4,00 | 5,00 | 3,00 | 2,50 | 2,00 | 2,00 | 1,00 | 1,50 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Divinyldimethicone/Dimethicone Copolymer (and) C12-13 Pareth-23 (and) C12-13 Pareth-3 | 2,30 | 2,30 | 2,30 | 2,30 | 2,30 | 2,30 | 2,30 | 2,30 | 2,30 |
| | Sodium Chloride | 2,00 | 2,00 | 2,00 | 2,00 | 2,00 | 2,00 | 2,00 | 2,00 | 2,00 |
| | Aqua | Qsto 100 | Qsto 100 | Qsto 100 | Qsto 100 | Qsto 100 | Qsto 100 | Qsto 100 | Qsto 100 | Qsto 100 |
| In vitro SPF measured according the method described | | 22,6 | 22,3 | 17,9 | 19,2 | 11,4 | 13,5 | 11,8 | 14,9 | 16,4 |
| UVA PF measured according the method described | | 4,8 | 6,3 | 6,2 | 6,5 | 6,7 | 7,8 | 7,6 | 8,4 | 8,5 |

Manufacturing instruction:

Part A is prepared by incorporating all ingredients, then stirred under moderate speed. Part C is prepared and heated to 60°C. At this temperature, part C is poured into part A under progressive stirring speed. Then the ingredient of part B is added separately.

| Example B18: W/ Si emulsion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| Part A | Tetrabutoxypropyl Trisiloxane | 0,90 | 0,90 | 0,90 | 0,90 | 0,90 | 0,90 | 0,90 | 0,90 | 0,90 |
| | Benzyl Laurate | 2,00 | 2,00 | 2,00 | 2,00 | 2,00 | 2,00 | 2,00 | 2,00 | 2,00 |
| | Cetyl dimethicone | 2,00 | 2,00 | 2,00 | 2,00 | 2,00 | 2,00 | 2,00 | 2,00 | 2,00 |

(continued)

| Example B18: W/ Si emulsion | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Polyglyceryl-4, isostearate, cetyl dimethicone copolyol and hexyl laurate | 6,00 | 6,00 | 6,00 | 6,00 | 6,00 | 6,00 | 6,00 | 6,00 | 6,00 |
| | polyglyceryl-3 dioleate | 0,60 | 0,60 | 0,60 | 0,60 | 0,60 | 0,60 | 0,60 | 0,60 | 0,60 |
| | Glyceryl tribehenate | 3,00 | 3,00 | 3,00 | 3,00 | 3,00 | 3,00 | 3,00 | 3,00 | 3,00 |
| | cyclomethicone | 8,85 | 8,85 | 8,85 | 8,85 | 8,85 | 8,85 | 8,85 | 8,85 | 8,85 |
| | Ethylhexyl Methoxycinnamate | 6,00 | 8,00 | 4,00 | 3,00 | 2,00 | 4,00 | 5,00 | 3,00 | 3,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Titanium Dioxide | 4,00 | 5,00 | 3,00 | 2,50 | 2,00 | 2,00 | 1,00 | 1,50 | 1,00 |
| Part B | Organic micro-particulate UV absorber of formula (3), (5) or (7) | 2,00 | 4,00 | 6,00 | 8,00 | 10,0 | 14,0 | 16,0 | 18,0 | 20,0 |
| Part C | Aqua | Qsto 100 | Qsto 100 | Qsto 100 | Qsto 100 | Qsto 100 | Qsto 100 | Qsto 100 | Qsto 100 | Qsto 100 |
| | Xanthan gum | 0,10 | 0,10 | 0,10 | 0,10 | 0,10 | 0,10 | 0,10 | 0,10 | 0,10 |
| | Disodium EDTA | 0,10 | 0,10 | 0,10 | 0,10 | 0,10 | 0,10 | 0,10 | 0,10 | 0,10 |
| | Sodium chloride | 1,00 | 1,00 | 1,00 | 1,00 | 1,00 | 1,00 | 1,00 | 1,00 | 1,00 |
| | Glycerin | 3,00 | 3,00 | 3,00 | 3,00 | 3,00 | 3,00 | 3,00 | 3,00 | 3,00 |
| | Disodium Phenyl Dibenzimidazole tetrasulfonate | 7,00 | 5,00 | 4,00 | 3,00 | 2,00 | 3,00 | 1,50 | 1,00 | 1,00 |
| | Terephthalidene Dicamphor sulfonic acid | 8,00 | 6,00 | 4,00 | 3,00 | 2,00 | 2,00 | 1,00 | 1,50 | 1,00 |
| Part D | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |
| | panthenol | 0,10 | 0,10 | 0,10 | 0,10 | 0,10 | 0,10 | 0,10 | 0,10 | 0,10 |
| | Sodium ascorbyl phosphate | 0,20 | 0,20 | 0,20 | 0,20 | 0,20 | 0,20 | 0,20 | 0,20 | 0,20 |
| | tocopheryl acetate | 0,50 | 0,50 | 0,50 | 0,50 | 0,50 | 0,50 | 0,50 | 0,50 | 0,50 |
| | Phytantriol | 0,30 | 0,30 | 0,30 | 0,30 | 0,30 | 0,30 | 0,30 | 0,30 | 0,30 |
| | Frag rance/preservative | q.s. | q.s. | q.s. | q.s. | q.s. | q.s. | q.s. | q.s. | q.s. |
| In vitro SPF measured according the method described | | 62,6 | 57,7 | 39,5 | 48,9 | 28,8 | 37,1 | 25,4 | 25,6 | 19,4 |
| UVA PF measured according the method described | | 29,6 | 25,7 | 20,4 | 27,5 | 18,3 | 22 | 15,5 | 17,4 | 14,8 |

Manufacturing instruction:

Part A is prepared by incorporating all ingredients, then stirred under moderate speed. Part C is prepared and heated to 60°C. At this temperature, part C is poured into part A under progressive stirring speed. Then the ingredient of part B is added separately and the part E is added under moderate stirring.

| Example B19: Sun Protection Gel (aqueous) | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Allantoin | 0,20 | 0,20 | 0,20 | 0,20 | 0,20 | 0,20 | 0,20 | 0,20 | 0,20 |
| | Sorbitol | 5,00 | 5,00 | 5,00 | 5,00 | 5,00 | 5,00 | 5,00 | 5,00 | 5,00 |
| | Aqua | Qsto 100 | Qsto 100 | Qsto 100 | Qsto 100 | Qsto 100 | Qsto 100 | Qsto 100 | Qsto 100 | Qsto 100 |
| | Disodium Phenyl Dibenzimidazole tetrasulfonate | 7,00 | 5,00 | 4,00 | 3,00 | 2,00 | 3,00 | 1,50 | 1,00 | 1,00 |
| | Phenylbenzimidazole sulfonic acid | 8,00 | 6,00 | 4,00 | 3,00 | 2,00 | 2,00 | 1,00 | 1,50 | 1,00 |
| | Zinc Oxide | 4,00 | 5,00 | 3,00 | 2,50 | 2,00 | 2,00 | 1,00 | 1,50 | 1,00 |
| | Organic micro-particulate UV absorber of formula (3), (5) or (7) | 2,00 | 4,00 | 6,00 | 8,00 | 10,0 | 14,0 | 16,0 | 18,0 | 20,0 |
| | Parfum | 0,30 | 0,30 | 0,30 | 0,30 | 0,30 | 0,30 | 0,30 | 0,30 | 0,30 |
| | PEG-35-Hydrogenated Castor Oil | 1,00 | 1,00 | 1,00 | 1,00 | 1,00 | 1,00 | 1,00 | 1,00 | 1,00 |
| | Carbomer | 1,50 | 1,50 | 1,50 | 1,50 | 1,50 | 1,50 | 1,50 | 1,50 | 1,50 |
| | Aqua | 36,1 | 36,1 | 36,1 | 36,1 | 36,1 | 36,1 | 36,1 | 36,1 | 36,1 |
| | Water, demineralized | 10,0 | 10,0 | 10,0 | 10,0 | 10,0 | 10,0 | 10,0 | 10,0 | 10,0 |
| | Citric Acid (and) Silver Citrate | 0,10 | 0,10 | 0,10 | 0,10 | 0,10 | 0,10 | 0,10 | 0,10 | 0,10 |
| | Sclerotium Gum | 0,10 | 0,10 | 0,10 | 0,10 | 0,10 | 0,10 | 0,10 | 0,10 | 0,10 |
| In vitro SPF measured according the method described | | 35,5 | 34,6 | 31,4 | 30,8 | 29,5 | 41,8 | 38 | 44,2 | 47,1 |
| UVA PF measured according the method described | | 20,8 | 19,3 | 15 | 13,4 | 11,7 | 14,1 | 11,3 | 12,9 | 12,3 |

All the ingredients are dispersed or solubilized inside the water phase under moderate stirring till an homogeneous gel appears. Then pH is checked.

| Example B20: Foundations: O/W forms | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Butylene Glycol | 6,50 | 6,50 | 6,50 | 6,50 | 6,50 | 6,50 | 6,50 | 6,50 | 6,50 |
| | Magnesium Aluminum Silicate | 0,50 | 0,50 | 0,50 | 0,50 | 0,50 | 0,50 | 0,50 | 0,50 | 0,50 |
| | Sodium Carboxymethylcellulose | 0,30 | 0,30 | 0,30 | 0,30 | 0,30 | 0,30 | 0,30 | 0,30 | 0,30 |
| | Xanthan Gum | 0,10 | 0,10 | 0,10 | 0,10 | 0,10 | 0,10 | 0,10 | 0,10 | 0,10 |
| | Triethanolamine | 1,00 | 1,00 | 1,00 | 1,00 | 1,00 | 1,00 | 1,00 | 1,00 | 1,00 |
| | Polysorbate 20 | 0,25 | 0,25 | 0,25 | 0,25 | 0,25 | 0,25 | 0,25 | 0,25 | 0,25 |

(continued)

| Example B20: Foundations: O/W forms | | | | | | | | | | |
|---|---|---|---|---|---|---|---|---|---|---|
| | | A | B | C | D | E | F | G | H | I |
| | INCI-Name | % w/w | % w/w | % w/w | % w/w | % w/w | % w/w | % w/w | % w/w | % w/w |
| | Sericite | 7,00 | 7,00 | 7,00 | 7,00 | 7,00 | 7,00 | 7,00 | 7,00 | 7,00 |
| | Iron oxides | 1,10 | 1,10 | 1,10 | 1,10 | 1,10 | 1,10 | 1,10 | 1,10 | 1,10 |
| | Spherical Silica | 1,00 | 1,00 | 1,00 | 1,00 | 1,00 | 1,00 | 1,00 | 1,00 | 1,00 |
| | Cetearyl octanoate | 10,0 | 10,0 | 10,0 | 10,0 | 10,0 | 10,0 | 10,0 | 10,0 | 10,0 |
| | Stearic Acid | 2,50 | 2,50 | 2,50 | 2,50 | 2,50 | 2,50 | 2,50 | 2,50 | 2,50 |
| | Glyceryl stearate | 2,00 | 2,00 | 2,00 | 2,00 | 2,00 | 2,00 | 2,00 | 2,00 | 2,00 |
| | Tridecyl trimellilate | 2,00 | 2,00 | 2,00 | 2,00 | 2,00 | 2,00 | 2,00 | 2,00 | 2,00 |
| | Bis-Ethylhexyloxyphenol | 4,00 | 3,00 | 2,00 | 6,00 | 2,50 | 3,00 | 1,50 | 2,00 | 1,00 |
| | Methoxyphenyl Triazine | | | | | | | | | |
| | Titanium Dioxide | 4,00 | 5,00 | 3,00 | 2,50 | 2,00 | 2,00 | 1,00 | 1,50 | 1,00 |
| | Polysilicone-15 | 6,00 | 8,00 | 4,00 | 3,00 | 2,00 | 4,00 | 5,00 | 3,00 | 3,00 |
| | Organic micro-particulate UV absorber of formula (3), (5) or (7) | 2,00 | 4,00 | 6,00 | 8,00 | 10,00 | 14,00 | 16,0 | 18,0 | 20,0 |
| | Water | qs to 100 | qs to 100 | qs to 100 | qs to 100 | qs to 100 | qs to 100 | qs to 100 | qs to 100 | qs to 100 |
| | Preservative | 0,55 | 0,55 | 0,55 | 0,55 | 0,55 | 0,55 | 0,55 | 0,55 | 0,55 |
| In vitro SPF measured according the method described | | 24,5 | 26,6 | 14,8 | 27,6 | 13,4 | 17,3 | 11,1 | 13,1 | 9,6 |
| UVA PF measured according the method described | | 11 | 11,2 | 9,1 | 15,8 | 10,5 | 12,5 | 9,9 | 11,6 | 10,1 |

Example B21: Si/W sun cream

| | | A | B | C | D | E | F | G | H | I |
|---|---|---|---|---|---|---|---|---|---|---|
| | INCI-name | % w/w | % w/w | % w/w | % w/w | % w/w | % w/w | % w/w | % w/w | % w/w |
| Part A | Cyclopentasiloxane (and) Dimethicone/Vinyl Dimethicone Cross polymer | 8,00 | 8,00 | 8,00 | 8,00 | 8,00 | 8,00 | 8,00 | 8,00 | 8,00 |
| | Dimethicone (and) Dimethicone/Vinyl Dimethicone Crosspolymer | 30,00 | 30,00 | 30,00 | 30,00 | 30,00 | 30,00 | 30,00 | 30,00 | 30,00 |
| | Cyclopentasiloxane | 10,00 | 10,00 | 10,00 | 10,00 | 10,00 | 10,00 | 10,0 | 10,0 | 10,0 |
| | Polysiloxane-15 | 6,00 | 8,00 | 4,00 | 3,00 | 2,00 | 4,00 | 5,00 | 3,00 | 3,00 |
| | Drometrizole trisloxane | 10,00 | 8,00 | 7,00 | 5,00 | 4,00 | 3,00 | 2,00 | 1,50 | 1,00 |
| | Bis-Ethylhexyloxyphenol Methoxyphenyl Triazine | 4,00 | 3,00 | 2,00 | 6,00 | 2,50 | 3,00 | 1,50 | 2,00 | 1,00 |
| | Ethylhexyl Methoxycinnamate | 6,00 | 8,00 | 4,00 | 3,00 | 2,00 | 4,00 | 5,00 | 3,00 | 3,00 |
| Part B | 1.3-Butylen Glycol | 3,00 | 3,00 | 3,00 | 3,00 | 3,00 | 3,00 | 3,00 | 3,00 | 3,00 |
| | Polyglyceryl-3 Disiloxane Dimethicone | 0,60 | 0,60 | 0,60 | 0,60 | 0,60 | 0,60 | 0,60 | 0,60 | 0,60 |
| | Polyglyceryl-3 Polydimethylsiloxyethyl Dimethicone | 0,30 | 0,30 | 0,30 | 0,30 | 0,30 | 0,30 | 0,30 | 0,30 | 0,30 |
| | Acrylamide/Sodium Acryloyldimethyltaurate Copolymer (and) Isohexadecane (and) Polysorbate 80 | 0,60 | 0,60 | 0,60 | 0,60 | 0,60 | 0,60 | 0,60 | 0,60 | 0,60 |
| | Organic micro-particulate UV absorber of formula (3), (5) or (7) | 2,00 | 4,00 | 6,00 | 8,00 | 10,00 | 14,00 | 16,0 | 18,0 | 20,0 |
| | Ammonium Acryloyldimethyltaurate/ VP Copolymer | 0,65 | 0,65 | 0,65 | 0,65 | 0,65 | 0,65 | 0,65 | 0,65 | 0,65 |
| | Sodium Chloride | 0,10 | 0,10 | 0,10 | 0,10 | 0,10 | 0,10 | 0,10 | 0,10 | 0,10 |
| | Aqua | Qs to 100 | Qs to 100 | Qs to 100 | Qs to 100 | Qs to 100 | Qs to 100 | Qs to 100 | Qs to 100 | Qs to 100 |
| In vitro SPF measured according the method described | | 39,2 | 37,7 | 23,3 | 35,5 | 16,9 | 24,2 | 20,5 | 16,9 | 13,8 |
| UVA PF measured according the method described | | 12,9 | 11,6 | 10,3 | 16,6 | 11,1 | 12,8 | 10,5 | 11,4 | 10,1 |

Manufacturing instruction:

Part A is prepared by incorporating all ingredients, then stirred under moderate speed. Part B is prepared and heated to 40°C. At this temperature, part A is poured into part B under progressive stirring speed till an homogeneous cream appears.

## Claims

1. A method of preparing a composition, comprising (a) a micronised insoluble organic UV absorber selected from the compounds of formula

(7) 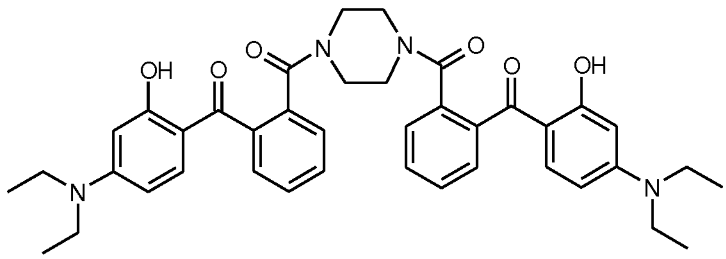 ,

which method comprises grinding the insoluble organic UV absorber, in coarse particle form, in a grinding apparatus, in the presence of a grinding aid selected from the groups

(b$_1$) Sodium Lauroyl Lactylate and
(b$_2$) Isocetyl isostearate, and/or glycol oleate.

2. A method according to claim 1 in which the micronised insoluble organic UV absorber so obtained has a mean particle size in the range of from 0.01 to 2.0μ.

3. A method according to any of the preceding claims in which the micronised insoluble organic UV absorber has been produced by grinding it, in coarse particulate form, in a grinding apparatus, until the insoluble organic UV absorber has been converted into micronised form.

4. A method according to claim 3 in which the grinding apparatus is a jet, ball, vibration or hammer mill.

5. A sunscreen composition comprising

(a) 0.1 to 25% by weight of a micronised insoluble organic UV absorber composition as prepared in claim 1 comprising a grinding aid selected from the groups
(b$_1$) Sodium Lauroyl Lactylate and
(b$_2$) Isocetyl isostearate, and/or glycol oleate; and

optionally a cosmetically acceptable carrier.

6. A composition according to claim 5 in which the micronised insoluble organic UV absorber is used together with one or more further UV absorbers which are conventionally used in cosmetic compositions for the protection of human skin against UV radiation.

7. A composition according claim 5 or 6, wherein

(c) a rheology modifier is additionally used.

8. A composition according to any of claims 5 to 7, wherein
component (c) is selected from Xanthan Gum, amorphous Silica and modified Starch.

9. Composition according to any of claims 5 to 8, wherein the rheology modifier (c) is used in a concentration of 0.1

to 10 % b.w. of the compositio

**Patentansprüche**

1. Verfahren zur Herstellung einer Zusammensetzung, umfassend (a) einen mikronisierten unlöslichen organischen UV-Absorber, der aus den Verbindungen der Formel

(7) 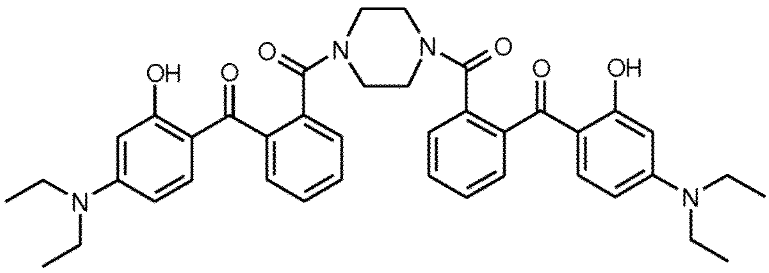 ,

ausgewählt ist, bei dem man den unlöslichen organischen UV-Absorber in grobteiliger Form in einer Mahlapparatur in Gegenwart einer Mahlhilfe, die aus den Gruppen

   ($b_1$) Natriumlauroyllactylat und
   ($b_2$) Isocetylisostearat und/oder Glykololeat ausgewählt ist, mahlt.

2. Verfahren nach Anspruch 1, bei dem der so erhaltene mikronisierte unlösliche organische UV-Absorber eine mittlere Teilchengröße im Bereich von 0,01 bis 2,0 μm aufweist.

3. Verfahren nach einem der vorhergehenden Ansprüche, bei dem der mikronisierte unlösliche organische UV-Absorber durch Mahlen in grobteiliger Form in einer Mahlapparatur bis zur Umwandlung des unlöslichen organischen UV-Absorbers in die mikronisierte Form hergestellt worden ist.

4. Verfahren nach Anspruch 3, bei dem es sich bei der Mahlapparatur um eine Düsen-, Kugel-, Vibrations- oder Hammermühle handelt.

5. Sonnenschutzzusammensetzung, umfassend

   (a) 0,1 bis 25 Gew.-% einer wie in Anspruch 1 hergestellten mikronisierten unlöslichen organischen UV-Absorber umfassenden Zusammensetzung, umfassend eine Mahlhilfe, die aus den Gruppen
   ($b_1$) Natriumlauroyllactylat und
   ($b_2$) Isocetylisostearat und/oder Glykololeat ausgewählt ist, und

   gegebenenfalls einen kosmetisch unbedenklichen Träger.

6. Zusammensetzung nach Anspruch 5, wobei der mikronisierte unlösliche organische UV-Absorber zusammen mit einem oder mehreren weiteren UV-Absorbern, die herkömmlicherweise in kosmetischen Zusammensetzungen zum Schutz menschlicher Haut gegen UV-Strahlung verwendet werden, verwendet wird.

7. Zusammensetzung nach Anspruch 5 oder 6, wobei (c) zusätzlich ein Rheologiemodifikator verwendet wird.

8. Zusammensetzung nach einem der Ansprüche 5 bis 7, wobei Komponente (c) aus Xanthangummi, amorphem Siliciumdioxid und modifizierter Stärke ausgewählt ist.

9. Zusammensetzung nach einem der Ansprüche 5 bis 8, wobei der Rheologiemodifikator (c) in einer Konzentration von 0,1 bis 10 Gew.-%, bezogen auf die Zusammensetzung, verwendet wird.

## Revendications

1. Méthode de préparation d'une composition, comprenant (a) un agent absorbant les UV organique insoluble et micronisé choisi parmi les composés de formule

(7) 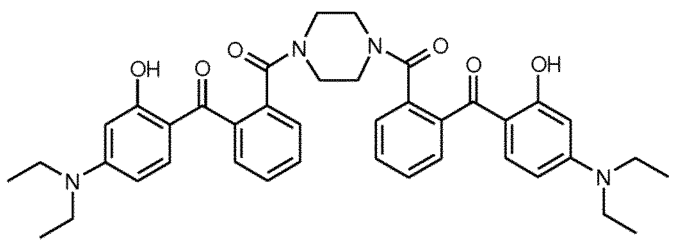 ,

laquelle méthode comprend le broyage de l'agent absorbant les UV organique insoluble, sous forme de particules grossières, dans un appareil de broyage, en présence d'un auxiliaire de broyage choisi dans le groupe constitué par

($b_1$) le lauroyl lactylate de sodium et
($b_2$) l'isostéarate d'isocétyle, et/ou l'oléate de glycol.

2. Méthode selon la revendication 1, dans laquelle l'agent absorbant les UV organique insoluble et micronisé ainsi obtenu possède une taille de particules moyenne dans la plage allant de 0,01 à 2,0 $\mu$m.

3. Méthode selon l'une quelconque des revendications précédentes, dans laquelle l'agent absorbant les UV organique insoluble et micronisé a été produit par son broyage, sous forme de particules grossières, dans un appareil de broyage, jusqu'à la conversion de l'agent absorbant les UV organique insoluble en forme micronisée.

4. Méthode selon la revendication 3, dans laquelle l'appareil de broyage est un broyeur à jet, à boulets, à vibration ou à marteaux.

5. Composition d'écran solaire, comprenant

(a) de 0,1 à 25% en poids d'une composition d'agent absorbant les UV organique insoluble et micronisé telle que préparée selon la revendication 1, comprenant un auxiliaire de broyage choisi dans le groupe constitué par
($b_1$) le lauroyl lactylate de sodium et
($b_2$) l'isostéarate d'isocétyle, et/ou l'oléate de glycol ; et

éventuellement un véhicule cosmétiquement acceptable.

6. Composition selon la revendication 5, dans laquelle l'agent absorbant les UV organique insoluble et micronisé est utilisé conjointement avec un ou plusieurs autres agents absorbants les UV qui sont utilisés de manière classique dans des compositions cosmétiques destinées à la protection de la peau humaine contre le rayonnement UV.

7. Composition selon la revendication 5 ou 6, dans laquelle

(c) un agent modificateur de rhéologie est en outre utilisé.

8. Composition selon l'une quelconque des revendications 5 à 7, dans laquelle le composant (c) est choisi parmi la gomme xanthane, la silice amorphe et un amidon modifié.

9. Composition selon l'une quelconque des revendications 5 à 8, dans laquelle l'agent modificateur de rhéologie (c) est utilisé selon une concentration allant de 0,1 à 10% en poids de la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 582189 A
- US 5338539 A
- US 5518713 A
- EP 613893 A
- EP 709080 A
- US 5601811 A
- WO 9700851 A
- JP 5086984 A
- JP 4330007 A
- DE 10011317
- EP 1133980 A
- EP 1046391 A
- EP 1167358 A
- WO 2004006878 A
- WO 0341675 A
- WO 2002039974 A
- DE 10229995
- EP 1371358 A
- EP 1371357 A
- EP 1371356 A
- DE 10138496
- US 20040247536 A


### Non-patent literature cited in the description


- Redox Mechanisms in Heterogeneous Photocatalysis. **SERPONE et al.** Electrochemistry in Colloids and Dispersions. VCH Publishers Inc, 1992
- *CHEMICAL ABSTRACTS,* 9004-73-3
- *CHEMICAL ABSTRACTS,* 61417-49-0
- *CHEMICAL ABSTRACTS,* 4086-70-8
- *CHEMICAL ABSTRACTS,* 74499-44-8
- *CHEMICAL ABSTRACTS,* 36861-47-9, 15087-24-8, 1641-17-4, 131-56-6, 131-55-5, 131-57-7, 4065-45-6, 131-54-4, 131-53-3, 56039-58-8, 70356-09-1, 118-56-9, 71617-10-2, 134-09-8, 89-46-3, 6197-30-4, 21245-02-3, 5466-77-3, 118-60-5, 88122-99-0, 150-13-0, 113010-52-9, 27503-81-7